# EUROPEAN PATENT APPLICATION

(11) **EP 2 103 607 A1**
(43) Date of publication of application: **23.09.2009**
(21) Application number: 07850604.5
(22) Date of filing: 14.12.2007
(51) Int. Cl.: C07D 335/02, A61K 31/382, A61P 3/04, A61P 3/06, A61P 3/08, A61P 3/10, A61P 7/10, A61P 9/04, A61P 9/10, A61P 9/12, A61P 13/12, A61P 19/06, A61P 25/00, A61P 27/02, A61P 31/04, A61P 43/00

(54) **1-PHENYL 1-THIO-D-GLUCITOL DERIVATIVE**

(30) Priority: 14.12.2006 JP 2006336517
(71) Applicant: TAISHO PHARMACEUTICAL CO., LTD, Tokyo 170-8633 (JP)
(72) Inventor: KAKINUMA, Hiroyuki, Tokyo 170-8633 (JP); OI, Takahiro, Tokyo 170-8633 (JP); HASHIMOTO, Yuko, Tokyo 170-8633 (JP); TAKAHASHI, Hitomi, Tokyo 170-8633 (JP); KOBASHI, Yohei, Tokyo 170-8633 (JP); IWATA, Yuki, Tokyo 170-8633 (JP); KAWABE, Kenichi, Tokyo 170-8633 (JP); TAKAHASHI, Masato, Tokyo 170-8633 (JP)
(74) Representative: HOFFMANN EITLE
(86) International application number: PCT/JP2007/074100
(87) International publication number: WO 2008/072726

(57) **Abstract**

A 1-phenyl 1-thio-D-glucitol compound represented by formula (I) or the like: 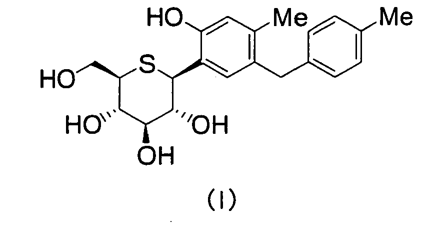 or a pharmaceutically acceptable salt thereof or a hydrate thereof, or a pharmaceutical preparation comprising the same as an active ingredient is useful as a novel type of prophylactic or therapeutic agent for diabetes, diabetes-related diseases or diabetic complications, which inhibits both SGLT1 and SGLT2 activities to achieve not only suppression of glucose absorption from the digestive tract but also excretion of urinary sugars.

## Description

### TECHNICAL FIELD

The present invention relates to pharmaceutical preparations, more particularly prophylactic or therapeutic agents for diabetes, diabetes-related diseases or diabetic complications, which have inhibitory activity against both sodium-dependent glucose transporter 1 (SGLT1) and sodium-dependent glucose transporter 2 (SGLT2) involved in glucose reabsorption in the kidney.

### BACKGROUND ART

When people suffer from diabetes, their fasting blood glucose levels reach 126 mg/dL or more. Even if fasting blood glucose levels fall within a normal range, some people exhibit postprandial blood glucose levels as high as 140 to 200 mg/dL and are diagnosed as having impaired glucose tolerance (hereinafter referred to as IGT). In recent years, it has been considered that the risk of cardiovascular disorders can be reduced by delaying the onset of diabetes from IGT. For example, the Da Qing IGT and Diabetes Study carried out in China in 1997 has reported that progression of IGT into Type II diabetes is significantly suppressed by diet and exercise (see Non-patent Document 1). As a case where medication is effective, an α-glucosidase inhibitor, acarbose, which inhibits sugar hydrolases to delay sugar absorption from the small intestine has been reported to suppress the development of Type II diabetes from IGT and further significantly suppress the onset of hypertension (see Non-patent Document 2).

In view of the foregoing, to suppress the onset of diabetes, it is important to control IGT by diet therapy, exercise therapy and medication. However, under the present circumstances, the number of diabetic patients increases with each passing year, and there has been a demand for novel therapeutic agents in response to various etiological factors and clinical conditions.

Diabetes is basically treated by diet therapy and exercise therapy; however, medication must be chosen when well-regulated lifestyle habits are difficult to maintain or when sufficient effect is not obtained by these therapies.

On the small intestinal epithelium, sodium-dependent glucose transporter 1 (SGLT1) is known to be expressed at a high frequency. SGLT1 serves depending upon sodium and plays a role in active transport of glucose or galactose in the small intestine (see Non-patent Document 3). Moreover, diabetic model rats are confirmed to have increased expression levels of SGLT1 mRNA and protein (see Non-patent Document 4). In recent years, some reports have been issued for synthesis of pyrazole derivatives which inhibit SGLT1 activity, suppress absorption of dietary glucose and thereby attempt to improve IGT (see Patent Documents 1 to 6).

Furthermore, sodium-dependent glucose transporter 2 (SGLT2) is expressed at a high frequency in the kidney. Glucose once filtered by the glomeruli is reabsorbed via SGLT2 (see Non-patent Document 5). When an SGLT2 inhibitor is administered to diabetic rats, sugar excretion into urine is facilitated to induce a hypoglycemic action. From this, an SGLT2-specific inhibitor has been considered as a target molecule serving as a novel therapeutic agent for diabetes (see Non-patent Document 6). In these circumstances, studies have been conducted on SGLT2 inhibitors, and various types of glucose derivatives have been provided (see Patent Documents 7 and 8).

Accordingly, if SGLT1 and SGLT2 activities can be inhibited simultaneously, a novel type of therapeutic agent for diabetes can be provided, which has not only postprandial hyperglycemia suppression action ascribed to SGLT1 inhibition but also progressive hypoglycemic action ascribed to SGLT2 inhibition.

Up to now, 1-thio-D-glucitol derivatives with relatively strong inhibitory activity against SGLT2 have been reported (see Patent Document 9); however, 1-phenyl 1-thio-D-glucitol derivatives strongly inhibiting both SGLT1 and SGLT2 have not yet been reported.

Patent Document 1: International Publication No. WO2002/098893
Patent Document 2: International Publication No. WO2004/014932
Patent Document 3: International Publication No. WO2004/018491
Patent Document 4: International Publication No. WO2004/019958
Patent Document 5: International Publication No. WO2005/121161
Patent Document 6: International Publication No. WO2004/050122
Patent Document 7: European Patent Publication No. 0850948
Patent Document 8: International Publication No. WO2001/068660
Patent Document 9: International Publication No. WO2006/073197
Non-patent Document 1: Pan XR, et al. Diabets Care, vol. 20, p. 537, 1997
Non-patent Document 2: J.-L. Chiasson, et al. Lancent, vol. 359, p. 2072, 2002
Non-patent Document 3: W. S. Lee, et al. J. Biol. Chem. vol. 269, p. 12032, 1994
Non-patent Document 4: Y. Fujita, et al. Diabetologia vol. 41, p. 1459, 1998
Non-patent Document 5: E. M. Wright, Am. J. Physiol. Renal. Physiol., vol. 280, p. F10, 2001
Non-patent Document 6: G. Toggenburger, et al. Biochem. Biophys. Acta., vol. 688, p. 557, 1982

### DISCLOSURE OF THE INVENTION

### PROBLEMS TO BE SOLVED BY THE INVENTION

The present invention aims to provide a novel type of therapeutic agent for diabetes, which inhibits both SGLT1 and SGLT2 activities to achieve not only suppression of glucose absorption from the digestive tract but also excretion of urinary sugars.

### MEANS FOR SOLVING THE PROBLEMS

To overcome the problems stated above, the inventors of the present invention have made extensive and intensive efforts to study substituents on the aglycon moiety of 1-thio-D-glucitol derivatives for their effect on SGLT inhibition. As a result, the inventors have found compounds (I) to (VII) which are designed to have certain types of substituents in combination and thereby strongly inhibit both SGLT1 and SGLT2 activities. This finding led to the completion of the present invention.

Namely, the present invention is directed to the following:
(1) a 1-phenyl 1-thio-D-glucitol compound represented by formula (I): 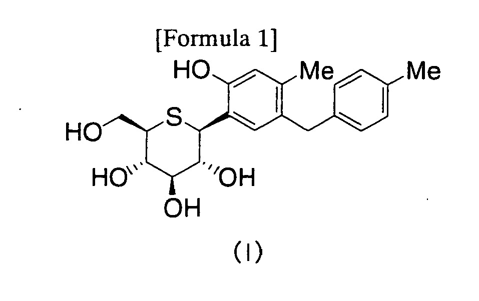 or a pharmaceutically acceptable salt thereof or a hydrate thereof;

(2) a 1-phenyl 1-thio-D-glucitol compound represented by formula (II): 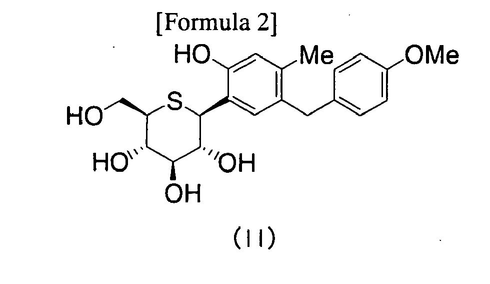 or a pharmaceutically acceptable salt thereof or a hydrate thereof;

(3) a 1-phenyl 1-thio-D-glucitol compound represented by formula (III): 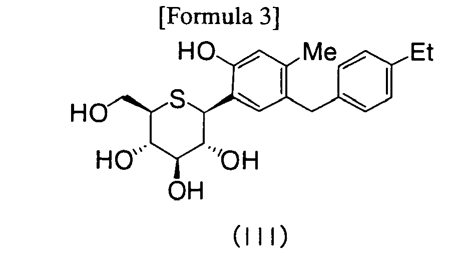 or a pharmaceutically acceptable salt thereof or a hydrate thereof;

(4) a 1-phenyl 1-thio-D-glucitol compound represented by formula (IV):

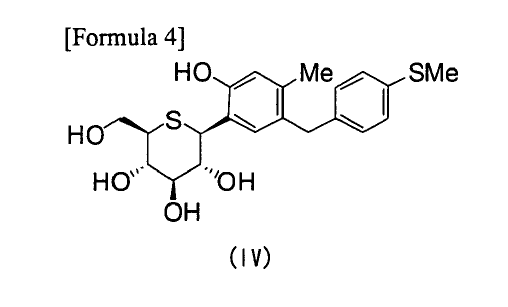

or a pharmaceutically acceptable salt thereof or a hydrate thereof;

(5) a 1-phenyl 1-thio-D-glucitol compound represented by formula (V): 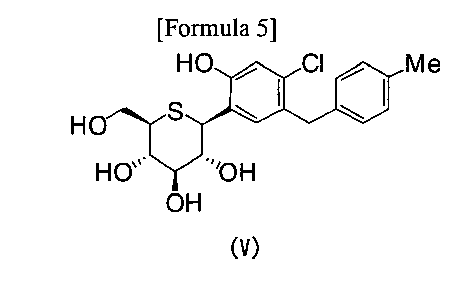 or a pharmaceutically acceptable salt thereof or a hydrate thereof;

(6) a 1-phenyl 1-thio-D-glucitol compound represented by formula (VI): 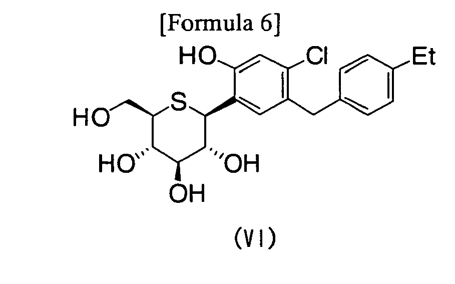 or a pharmaceutically acceptable salt thereof or a hydrate thereof; and

(7) a 1-phenyl 1-thio-D-glucitol compound represented by formula (VII):

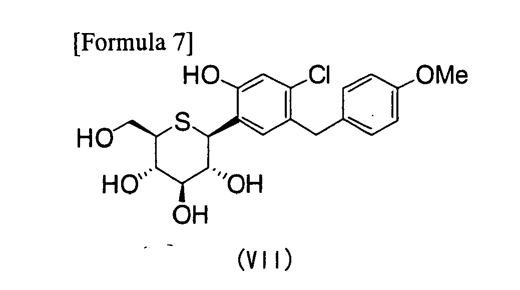

or a pharmaceutically acceptable salt thereof or a hydrate thereof.

In another embodiment, the present invention is directed to a pharmaceutical preparation, which comprises a compound represented by any of the above formulae (I) to (VII) or a pharmaceutically acceptable salt thereof or a hydrate thereof as an active ingredient.

In yet another embodiment, the present invention is directed to a pharmaceutical preparation, which comprises a compound represented by any of the above formulae (I) to (VII) or a pharmaceutically acceptable salt thereof or a hydrate thereof as an active ingredient, wherein the pharmaceutical preparation is an inhibitor of both sodium-dependent glucose transporter 1 activity and sodium-dependent glucose transporter 2 activity.

In yet another embodiment, the present invention is directed to a prophylactic or therapeutic agent for diabetes, diabetes-related diseases or diabetic complications, which comprises a compound represented by any of the above formulae (I) to (VII) or a pharmaceutically acceptable salt thereof or a hydrate thereof as an active ingredient.

### ADVANTAGES OF THE INVENTION

1-Thio-D-glucitol derivatives whose aglycon moiety was modified to have certain types of substituents in combination were found to inhibit both SGLT1 and SGLT2 activities. These compounds also showed an excellent hypoglycemic effect.

### BEST MODE FOR CARRYING OUT THE INVENTION

As used herein, the term "pharmaceutically acceptable salt" is intended to mean, for example, a salt with an alkali metal, an alkaline earth metal, ammonium or an alkylammonium, or a salt with a mineral acid or an organic acid. Examples include a sodium salt, a potassium salt, a calcium salt, an ammonium salt, an aluminum salt, a triethylammonium salt, an acetate salt, a propionate salt, a butyrate salt, a formate salt, a trifluoroacetate salt, a maleate salt, a tartrate salt, a citrate salt, a stearate salt, a succinate salt, an ethylsuccinate salt, a lactobionate salt, a gluconate salt, a glucoheptate salt, a benzoate salt, a methanesulfonate salt, an ethanesulfonate salt, a 2-hydroxyethanesulfonate salt, a benzenesulfonate salt, a p-toluenesulfonate salt, a lauryl sulfate salt, a malate salt, an aspartate salt, a glutamate salt, an adipate salt, a salt with cysteine, a salt with N-acetylcysteine, a hydrochloride salt, a hydrobromide salt, a phosphate salt, a sulfate salt, a hydroiodide salt, a nicotinate salt, an oxalate salt, a picrate salt, a thiocyanate salt, an undecanoate salt, a salt with an acrylate polymer and a salt with a carboxyvinyl polymer.

The term "hydrate" is intended to mean a pharmaceutically acceptable hydrate of any compound of the present invention or a salt thereof. When exposed to air or recrystallized, the compounds of the present invention or salts thereof may absorb moisture to thereby have adsorbed water or form hydrates. Such hydrates also fall within the scope of the present invention.

How to prepare the compounds (I) to (VII) of the present invention will be illustrated below.

Preparation Procedure 1

In the scheme shown below, R^{A} represents a methyl group, an ethyl group, a methoxy group or a methylthio group, R^{B} represents a methyl group or a chloro group, and R^{C} represents a benzyl group or an allyl group.

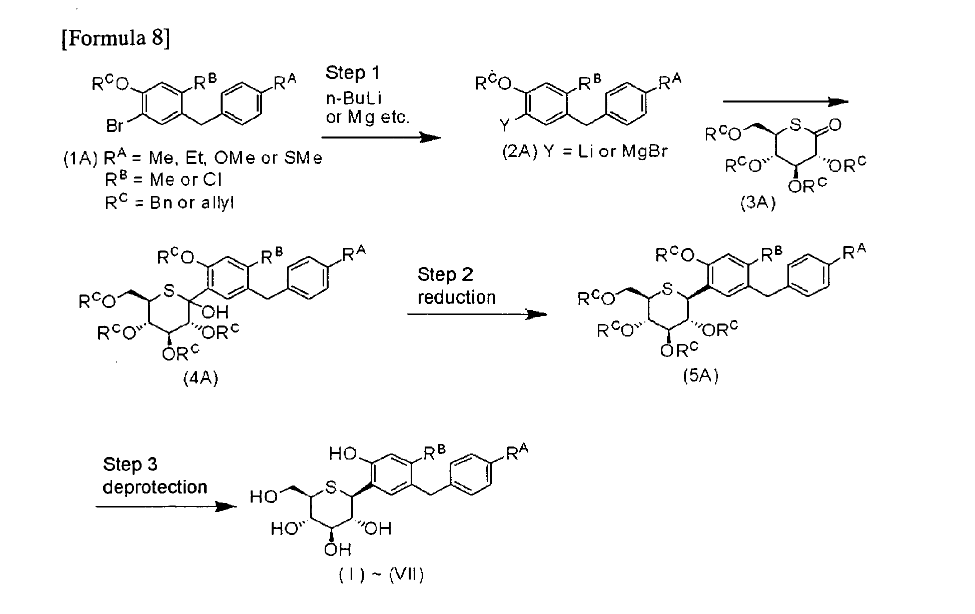

### (1) Step 1

Compound (1A) may be prepared according to International Publication No. WO2006/073197. Alternatively, it may also be prepared according to the reference examples described later. Compound (1A) may be treated with an organometallic reagent (e.g., n-butyllithium, sec-butyllithium, tert-butyllithium) to prepare lithium reagent (2A). Examples of a solvent available for use in the reaction include tetrahydrofuran, diethyl ether, and toluene. The reaction temperature ranges from -78°C to room temperature, preferably from -78°C to -25°C. Alternatively, magnesium powder may be used to prepare Grignard reagent (2A). Examples of a solvent available for use in the reaction include tetrahydrofuran, diethyl ether, and diglyme. In some cases, iodine or 1,2-dibromoethane may be used as an activator for magnesium.

Next, to compound (2A), thiolactone (3A) may be added to obtain compound (4A). The preferred temperature at which thiolactone (3A) is added is -78°C to -25°C when (2A) is a lithium reagent, while it is -15°C to 25°C when (2A) is a Grignard reagent.

### (2) Step 2 (Reduction)

Compound (4A) and Et₃SiH, i-Pr₃SiH, t-BuMe₂SiH or Ph₂SiHCl may be reacted in the presence of an acid to synthesize compound (5A). Examples of an acid available for use in this reaction include BF₃·Et₂O, CF₃COOH, MeSO₃H, and InCl₃. Examples of a solvent include chloroform, dichloromethane, acetonitrile or mixed solvents thereof, and preferred are mixed solvents with acetonitrile such as acetonitrile-chloroform, acetonitrile-dichloromethane, etc. The reaction temperature in this case ranges from -60°C to 25°C, preferably from -30°C to 25°C.

### (3) Step 3 (Deprotection)

Compound (5A) obtained above, in which R^{C} is a benzyl group, may be catalytically hydrogenated using a catalyst (e.g., palladium on activated carbon, palladium hydroxide, or platinum-palladium on activated carbon) under a hydrogen atmosphere to cause debenzylation, thereby giving the compound of the present invention. Above all, palladium on activated carbon or palladium hydroxide is preferred as a catalyst. Examples of a solvent available for use in this reaction include methanol, ethanol, isopropanol, ethyl acetate, acetic acid, and mixed solvents thereof. The reaction temperature ranges from room temperature to reflux temperature, with room temperature being preferred.

During debenzylation, it is also possible to use an acid such as BCl₃, BCl₃·Me₂S, BBr₃, AlCl₃, CF₃COOH or TfOH. Examples of a solvent available for use in this reaction include chloroform, dichloromethane, acetonitrile, diethyl ether, tetrahydrofuran, dimethyl sulfide, and anisole. Above all, it is preferable to use CF₃COOH, TfOH and ethanedithiol in dimethyl sulfide. The reaction temperature desirably ranges from -78°C to 40°C.
Compound (5A), in which R^{C} is an allyl group (-CH₂CH=CH₂), may be treated with potassium tert-butoxide in dimethyl sulfoxide to cause isomerization (-CH=CHCH₃), followed by removal of the isomerized groups using hydrochloric acid or HgCl₂/HgO. Alternatively, these groups may be removed in the presence of an organic acid (e.g., acetic acid, p-toluenesulfonic acid hydrate, N,N'-dimethylbarbituric acid) by using Pd(PPh₃)₄, PdCl₂, palladium on activated carbon, etc. Examples of a solvent available for use in this reaction include acetonitrile, diethyl ether, and tetrahydrofuran. The reaction temperature desirably ranges from 25°C to 100°C.

### Preparation Procedure 2

Compound (5A) can also be prepared in the following manner. In the scheme shown below, the symbols are as defined above.

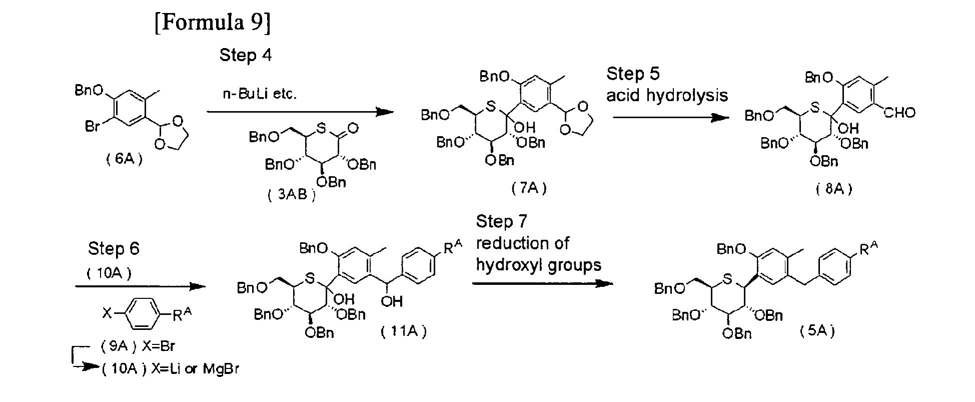

### (4) Step 4

In the same manner as shown in Step 1, intermediate compound (6A) may be treated with an organometallic reagent (e.g., n-butyllithium, sec-butyllithium, tert-butyllithium) to prepare an aryllithium reagent. To this reagent, thiolactone (3AB) may be added to obtain compound (7A). Examples of a solvent available for use in the reaction include tetrahydrofuran, diethyl ether, and toluene. The reaction temperature ranges from -78°C to room temperature, preferably from -78°C to -25°C.

### (5) Step 5 (Acid hydrolysis)

The acetal group in compound (7A) may be hydrolyzed with hydrochloric acid, p-toluenesulfonic acid monohydrate or the like to prepare compound (8A). Examples of a solvent preferred for this purpose include tetrahydrofuran, ethanol, methanol, water, or mixed solvents thereof. The reaction temperature ranges from 4°C to 60°C, with room temperature being preferred. The reaction time will vary depending on the reaction temperature, but it ranges from 1 to 24 hours.

### (6) Step 6

4-Substituted toluene (9A) may be treated with n-butyllithium, sec-butyllithium, tert-butyllithium or the like to prepare phenyllithium reagent compound (10A). Examples of a solvent available for use in the reaction include tetrahydrofuran, diethyl ether, and toluene. The reaction temperature ranges from -78°C to room temperature, preferably from -78°C to -25°C. The reaction time preferably ranges from 5 to 30 minutes. Alternatively, metal magnesium may be used to prepare Grignard reagent (10A). Examples of a solvent available for use in the reaction include tetrahydrofuran, diethyl ether, and diglyme. Next, reagent (10A) and compound (8A) may be reacted to prepare compound (11A).

### (7) Step 7 (Reduction)

Compound (11A) may be reacted with Et₃SiH, i-Pr₃SiH, t-BuMe₂SiH or Ph₂SiHCl in the presence of an acid to synthesize compound (5A). Examples of an acid available for use in this reaction include BF₃·Et₂O, CF₃COOH, and InCl₃. Examples of a solvent include chloroform, dichloromethane, acetonitrile or mixed solvents thereof, and preferred are mixed solvents with acetonitrile such as acetonitrile-chloroform, acetonitrile-dichloromethane, etc. The reaction temperature in this case ranges from -60°C to 25°C, preferably -30°C to 0°C.

In a particularly preferred procedure, the highly reactive hemiacetal moiety is reduced in acetonitrile solvent using about 1 equivalent of an acid at a reaction temperature of -15°C to -5°C. Next, the reaction temperature is elevated to 0°C to 5°C and about 1 equivalent of an acid is further added to reduce the hydroxyl group at the benzyl position.

### Preparation of thiolactone (3A)

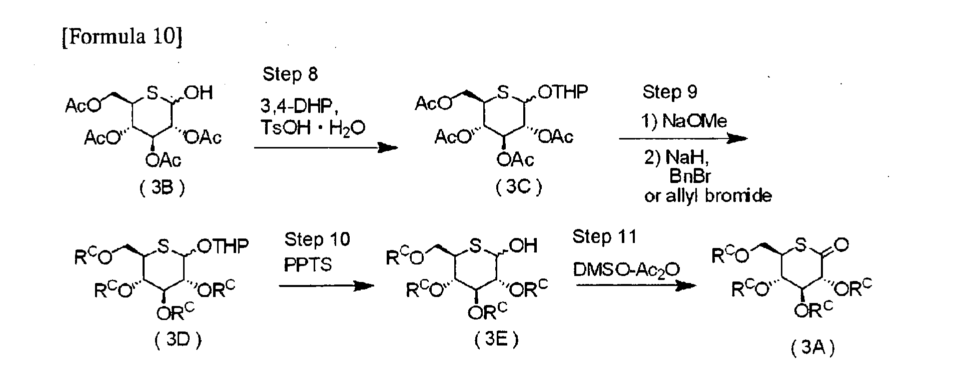

Compound (3A) can be synthesized by reference to Yuasa, H., et al. J. Chem. Soc. Perkin Trans. 1, p. 2763, 1990. Alternatively, it may be synthesized according to the scheme shown above.

### (8) Step 8

The hydroxyl group at the 1-position of compound (3B) (which can be prepared by reference to International Publication No. WO04/106352) is protected with a protecting group which is resistant to basic conditions and is deprotectable under neutral or acidic conditions. For example, the hydroxyl group may be protected with a tetrahydropyranyl (THP) group using 3,4-dihydro-2H-pyran (3,4-DHP) and p-toluenesulfonic acid monohydrate to synthesize compound (3C). Examples of a solvent preferred for this purpose include tetrahydrofuran, diethyl ether, and chloroform.

### (9) Step 9

The acetoxy groups as the protecting group may be removed using a base such as sodium methoxide, sodium hydroxide, lithium hydroxide, potassium carbonate, cesium carbonate or triethylamine in a solvent such as methanol, ethanol or aqueous methanol, followed by treatment with benzyl bromide, benzyl chloride, allyl bromide or the like using an appropriate base to give compound (3D). Examples of a base include triethylamine, N-ethyl-N,N-diisopropylamine, pyridine, potassium carbonate, calcium carbonate, cesium carbonate, sodium hydroxide, potassium hydroxide, sodium hydride, sodium methoxide and potassium tert-butoxide, with potassium carbonate, calcium carbonate, cesium carbonate, sodium hydroxide, potassium hydroxide and sodium hydride being preferred. Examples of a solvent available for use in this reaction include N,N-dimethylformamide, dimethyl sulfoxide, tetrahydrofuran, dioxane, and dimethoxyethane. The reaction temperature preferably ranges from -20°C to 25°C.

### (10) Step 10

Next, the protecting group at the 1-position may be removed to give compound (3E). For example, compound (3D) may be treated in methanol or ethanol with p-toluenesulfonic acid or pyridinium p-toluenesulfonate to remove the THP group.

### (11) Step 11

In the final step, compound (3E) may be treated with an appropriate oxidizing agent to prepare thiolactone (3A). Examples of an oxidizing agent preferred for use in this reaction include dimethyl sulfoxide-acetic anhydride, Dess-Martin periodinane, and IBX. The reaction temperature ranges from 0°C to 40°C.

The compounds of the present invention inhibit both SGLT1 and SGLT2 activities to improve IGT through not only suppression of glucose absorption from the digestive tract but also excretion of urinary sugars, thereby allowing prevention or treatment of diabetes.

Thus, the compounds of the present invention can be used as active ingredients in SGLT1 and SGLT2 inhibitors or in prophylactic or therapeutic agents for diabetes, diabetes-related diseases and diabetic complications.

As used herein, the term "diabetes" encompasses type I diabetes, type II diabetes, and other types of diabetes with specific etiology.

As used herein, the term "diabetes-related diseases" includes obesity, hyperinsulinemia, abnormal carbohydrate metabolism, hyperlipidemia, hypercholesterolemia, hypertriglyceridemia, abnormal lipid metabolism, hypertension, congestive heart failure, edema, hyperuricemia and gout.

As used herein, the term "diabetic complications" can be classified into acute complications and chronic complications.

"Acute complications" include hyperglycemia (e.g., ketoacidosis), infections (e.g., skin, soft tissue, biliary system, respiratory system and urinary tract infections), etc.

"Chronic complications" include microangiopathy (e.g., nephropathy, retinopathy), arteriosclerosis (e.g., atherosclerosis, heart infarction, brain infarction, lower extremity arterial occlusion), neuropathy (e.g., sensory nerves, motor nerves, autonomic nerves), foot gangrene, etc.

Major complications are diabetic retinopathy, diabetic nephropathy and diabetic neuropathy.

The dosage of the compounds of the present invention will vary depending on the disease or symptom to be treated, body weight, age, sex, the route of administration, etc.
The daily dosage for adults is 0.1 to 1000 mg/kg body weight, preferably 0.1 to 200 mg/kg body weight, and more preferably 0.1 to 10 mg/kg body weight, given as a single dose or in divided doses.

With the aim of enhancing their action or reducing their dosage, the compounds of the present invention may also be used in combination with any drug (hereinafter abbreviated as a partner drug) which depends on a different mechanism of action other than inhibition of SGLT1 or SGLT2 activity, such as a therapeutic agent for diabetes, a therapeutic agent for diabetic complications, an antihyperlipidemic agent, a hypotensive agent, an antiobesity agent, a diuretic and/or an antithrombotic agent. In this case, there is no limitation on the timing of administering the compound of the present invention and its partner drug(s). They may be administered to a target, either simultaneously or with a time interval(s). The compound of the present invention and its partner drug(s) may be administered as two separate formulations containing the respective active ingredients or may be administered as a single formulation containing both active ingredients. The dosage of partner drugs may be selected as appropriate on the basis of their clinically used doses. Likewise, the ratio between the compound of the present invention and its partner drug(s) may be selected as appropriate for the target to be administered, the route of administration, the disease or symptom to be treated, the combination of drugs, etc. For example, when the target to be administered is human, partner drug(s) may be used in an amount of 0.01 to 100 parts by weight relative to 1 part by weight of the compound of the present invention.

Examples of a therapeutic agent for diabetes include insulin formulations (e.g., animal insulin formulations extracted from bovine and swine pancreases; human insulin formulations synthesized by genetic engineering techniques using E. coli or yeast cells; insulin zinc; protamine insulin zinc; insulin fragments or derivatives (e.g., INS-1), oral insulin formulations), insulin resistance-improving agents (e.g., pioglitazone or a salt thereof (preferably hydrochloride salt), rosiglitazone or a salt thereof (preferably maleate salt), Rivoglitazone (CS-011) (R-119702), Sipoglitazar (TAK-654), Metaglidasen (MBX-102), Naveglitazar (LY-519818), MX-6054, Balaglitazone (NN-2344), T-131 (AMG131), PPARγ agonists, PPARγ antagonists, PPARγ/α dual agonists, α-glucosidase inhibitors (e.g., voglibose, acarbose, miglitol, emiglitate), biguanides (e.g., phenformin, metformin, buformin or salts thereof (e.g., hydrochloride salt, fumarate salt, succinate salt)), insulin secretion stimulators (sulfonylureas (e.g., tolbutamide, glibenclamide, gliclazide, chlorpropamide, tolazamide, acetohexamide, glyclopyramide, glimepiride, glipizide, glybuzole), repaglinide, senaglinide, nateglinide, mitiglinide or calcium salt hydrates thereof), GPR40 agonists, GPR40 antagonists, GLP-1 receptor agonists (e.g., GLP-1, GLP-1MR, Liraglutide (NN-2211), Exenatide (AC-2993) (exendin-4), Exenatide LAR, BIM51077, Aib(8,35)Hglp-1(7,37)NH2, CJC-1131, AVE0010, GSK-716155), amylin agonists (e.g., pramlintide), phosphotyrosine phosphatase inhibitors (e.g., sodium vanadate), dipeptidyl peptidase IV inhibitors (e.g., compounds described in WO02/038541, NVP-DPP-278, PT-100, P32/98, Vildagliptin (LAF-237), P93/01, Sitagliptin (MK-431), Saxagliptin (BMS-477118), SYR-322, MP-513, T-6666, GRC-8200), β3 agonists (e.g., AJ-9677, AZ40140), gluconeogenesis inhibitors (e.g., glycogen phosphorylase inhibitors, glucose-6-phosphatase inhibitors, glucagon antagonists, fructose-1,6-bisphosphatase inhibitors), SGLT (sodium-glucose cotransporter) inhibitors (e.g., compounds described in WO04/014931, WO04/089967 and WO06/073197, T-1095, Sergliflozin (GSK-869682), GSK-189075, KGT-1251, KGT-1681, KGA-2727, BMS-512148, AVE2268, SAR7226), 11β-hydroxysteroid dehydrogenase inhibitors (e.g., compounds described in WO06/051662, BVT-3498, INCB13739), GPR119 agonists (e.g., PSN-632408, APD-668), adiponectin or agonists thereof, IKK inhibitors (e.g., AS-2868), AMPK activators, leptin resistance-improving agents, somatostatin receptor agonists, glucokinase activators (e.g., Ro-28-1675), pancreatic lipase inhibitors (e.g., orlistat, ATL-962), and DGAT-1 inhibitors.

Examples of a therapeutic agent for diabetic complications include aldose reductase inhibitors (e.g., tolrestat, epalrestat, zenarestat, zopolrestat, minalrestat, fidarestat, CT-112), neurotrophic factors and enhancers thereof (e.g., NGF, NT-3, BDNF, neurotrophin production/secretion stimulators), neuranagenesis stimulators (e.g., Y-128), PKC inhibitors (e.g., ruboxistaurin mesylate; LY-333531)), AGE inhibitors (e.g., ALT946, pimagedine, pyratoxathine, N-phenacylthiazolium bromide (ALT766), ALT-711, EXO-226, Pyridorin, pyridoxamine), active oxygen scavengers (e.g., thioctic acid), cerebral vasodilators (e.g., tiapride, mexiletine), somatostatin receptor agonists (e.g., BIM23190), and apoptosis signal-regulating kinase-1 (ASK-1) inhibitors.

Examples of an antihyperlipidemic agent include statin compounds (e.g., pravastatin, simvastatin, lovastatin, atorvastatin, fluvastatin, itavastatin, rosuvastatin, pitavastatin or salts thereof (e.g., sodium salt, calcium salt)), squalene synthase inhibitors (e.g., TAK-475), fibrate compounds (e.g., bezafibrate, clofibrate, simfibrate, clinofibrate), ACAT inhibitors (e.g., Avasimibe, Eflucimibe), anion exchange resins (e.g., cholestyramine), probucol, nicotinic acid drugs (e.g., nicomol, niceritrol), ethyl icosapentate, plant sterols (e.g., soysterol, γ-oryzanol), CETP inhibitors (e.g., Torcetrapib, JTT-705, JTT-302, FM-VP4), and cholesterol absorption inhibitors (e.g., Ezetimibe).

Examples of a hypotensive agent include angiotensin-converting enzyme inhibitors (e.g., captopril, enalapril, delapril), angiotensin II antagonists (e.g., candesartan cilexetil, losartan, eprosartan, valsartan, telmisartan, irbesartan, tasosartan, azilsartan (TAK-536)), calcium antagonists (e.g., manidipine, nifedipine, amlodipine, efonidipine, nicardipine), potassium channel openers (e.g., levcromakalim, L-27152, AL0671, NIP-121), and clonidine.

Examples of an antiobesity agent include central antiobesity agents (e.g., dexfenfluramine, fenfluramine, phentermine, sibutramine, amfepramone, dexamfetamine, mazindol, phenylpropanol amine, clobenzorex; MCH receptor antagonists (e.g., compounds described in WO06/035967, SB-568849; SNAP-7941, T-226296); neuropeptide Y antagonists (e.g., CP-422935); cannabinoid receptor antagonists (e.g., Rimonabant (SR-141716), SR-147778); ghrelin antagonists; 11β-hydroxysteroid dehydrogenase inhibitors (e.g., BVT-3498, INCB13739)), pancreatic lipase inhibitors (e.g., orlistat, ATL-962), DGAT-1 inhibitors, β3 agonists (e.g., AJ-9677, AZ40140), peptidic anorectics (e.g., leptin, CNTF (ciliary neurotrophic factor)), cholecystokinin agonists (e.g., lintitript, FPL-15849), and feeding deterrents (e.g., P-57).

Examples of a diuretic include xanthine derivatives (e.g., theobromine sodium salicylate, theobromine calcium salicylate), thiazide formulations (e.g., ethiazide, cyclopenthiazide, trichlormethiazide, hydrochlorothiazide, hydroflumethiazide, bentylhydrochlorothiazide, penflutizide, polythiazide, methyclothiazide), anti-aldosterone formulations (e.g., spironolactone, triamterene), carbonic anhydrase inhibitors (e.g., acetazolamide), chlorobenzenesulfonamide formulations (e.g., chlorthalidone, mefruside, indapamide), azosemide, isosorbide, ethacrynic acid, piretanide, bumetanide, and furosemide.

Examples of an antithrombotic agent include heparin (e.g., heparin sodium, heparin calcium, dalteparin sodium, AVE-5026), warfarin (e.g., warfarin potassium), antithrombins (e.g., argatroban, Ximelagatran, Dabigatran, Odiparcil, Lepirudin, bivalirudin, Desirudin, ART-123, Idraparinux, SR-123781, AZD-0837, MCC-977, TGN-255, TGN-167, RWJ-58436, LB-30870, MPC-0920, Pegmusirudin, Org-426751), thrombolytic agents (e.g., urokinase, tisokinase, alteplase, nateplase, monteplase, pamiteplase), platelet aggregation inhibitors (e.g., ticlepidine hydrochloride, cilostazol, ethyl icosapentate, beraprost sodium, sarpogrelate hydrochloride), factor Xa inhibitors (e.g., Fondaparinux, BAY-59-7939, DU-176b, YM-150, SR-126517, Apixaban, Razaxaban, LY-517717, MLN-102, Octaparine, Otamixaban, EMD-503982, TC-10, CS-3030, AVE-3247, GSK-813893, KFA-1982), and plasma carboxypeptidase B (also known as activated thrombin-activatable fibrinolysis inhibitor [TAFIa]) inhibitors (e.g., AZD-9684, EF-6265, MN-462).

The compounds of the present invention can be administered systemically or topically by the oral or parenteral route in the form of pharmaceutical preparations.

When the compounds of the present invention are provided in the form of pharmaceutical preparations, various types of dosage forms such as solids and solutions may be selected as appropriate. In this case, a pharmaceutically acceptable carrier(s) may also be incorporated. Examples of such a carrier include commonly used excipients, extenders, binders, disintegrating agents, coating agents, sugar-coating agents, pH adjustors, solubilizers, or aqueous or non-aqueous solvents. The compounds of the present invention and these carriers may be formulated into tablets, pills, capsules, granules, powders, solutions, emulsions, suspensions, injections or other dosage forms.

### EXAMPLES

The present invention will be further described in more detail by way of the following reference examples, examples and test examples.

### Reference Example 1

Preparation of 2,3,4,6-tetra-O-benzyl-5-thio-D-glucono-1,5-lactone (compound (3AB))

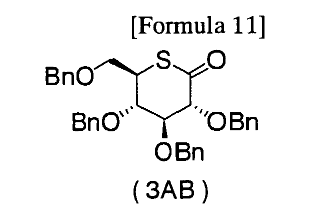

### (1) Preparation of tetrahydro-2H-pyran-2-yl 2,3,4,6-tetra-O-acetyl-5-thio-D-glucopyranose

To a solution of 2,3,4,6-tetra-O-acetyl-5-thio-D-glucopyranose (2.0 g, 5.49 mmol) in chloroform (40 mL), 3,4-dihydro-2H-pyran (1.5 mL, 16.5 mmol) and p-toluenesulfonic acid monohydrate (104 mg, 0.549 mmol) were added and stirred at room temperature for 1 hour. After addition of saturated aqueous sodium bicarbonate, the reaction mixture was extracted with chloroform, and the organic layer was washed with brine and then dried over anhydrous magnesium sulfate. After filtering off the desiccant, the solvent was distilled off under reduced pressure and the resulting residue was purified by silica gel column chromatography (hexane:ethyl acetate = 1:1) to give the titled compound (2.56 g) as a light-yellow amorphous substance.

### (2) Preparation of tetrahydro-2H-pyran-2-yl 2,3,4,6-tetra-O-benzyl-5-thio-D-glucopyranose

Next, to a solution of tetrahydro-2H-pyran-2-yl 2,3,4,6-tetra-O-acetyl-5-thio-D-glucopyranose (2.5 g) in methanol (40 mL), a 25 wt% methanol solution of sodium methoxide (0.11 mL, 0.55 mmol) was added and stirred for 3 hours. A small amount of dry ice was added to neutralize the reaction mixture, which was then concentrated. The resulting residue was dissolved in N,N-dimethylformamide (20 mL). This solution was added dropwise to a suspension of sodium hydride (1.3 g, 32.9 mmol; 60% in oil) in N,N-dimethylformamide (4 mL) under ice cooling. The reaction mixture was stirred at room temperature for 20 minutes and then cooled to 4°C, followed by addition of benzyl bromide (5.6 g, 32.9 mmol). The reaction mixture was stirred at room temperature for 12 hours and methanol (5 mL) was added thereto, followed by stirring for 30 minutes. After addition of ice-cold water, the reaction mixture was extracted with ethyl acetate, and the organic layer was washed with brine and then dried over anhydrous magnesium sulfate. After filtering off the desiccant, the solvent was distilled off under reduced pressure and the resulting residue was purified by silica gel column chromatography (hexane:ethyl acetate = 6:1) to give the titled compound (3.36 g, 96%, 2 steps).

### (3) Preparation of 2,3,4,6-tetra-O-benzyl-5-thio-D-glucopyranose

A mixture of tetrahydro-2H-pyran-2-yl 2,3,4,6-tetra-O-benzyl-5-thio-D-glucopyranose (3.30 g, 5.15 mmol), pyridinium p-toluenesulfonate (518 mg, 2.06 mmol) and ethanol (58 mL) was stirred at 80°C for 2 hours. The reaction mixture was cooled to room temperature and the solvent was concentrated. The resulting residue was dissolved in ethyl acetate. This solution was washed with saturated aqueous sodium bicarbonate and brine, and then dried over anhydrous magnesium sulfate. After filtering off the desiccant, the residue was purified by silica gel column chromatography (hexane:ethyl acetate = 3:1) to give the titled compound (2.89 g, quant.) as a colorless crystal.
¹³C NMR (125 MHz, CHLOROFORM-d) δ 41.3, 67.8, 71.6, 73.0, 73.2, 75.6, 76.2, 81.9, 82.9, 84.4, 127.5, 127.7, 127.8, 127.9, 128.0, 128.3, 128.4, 128.5, 137.8, 138.3, 138.8.

### (4) Preparation of 2,3,4,6-tetra-O-benzyl-5-thio-D-glucono-1,5-lactone

A mixture of 2,3,4,6-tetra-O-benzyl-5-thio-D-glucopyranose (2.82 g, 5.07 mmol), dimethyl sulfoxide (47 mL) and acetic anhydride (39 mL) was stirred at room temperature for 12 hours. After addition of ice-cold water, the reaction mixture was extracted with ethyl acetate. The organic layer was washed with water, saturated aqueous sodium bicarbonate and brine, and then dried over anhydrous magnesium sulfate. After filtering off the desiccant, the solvent was distilled off under reduced pressure and the resulting residue was purified by silica gel column chromatography (hexane:ethyl acetate = 6:1) to give the titled compound (2.3 g, 82%) as a colorless oil.
¹H NMR (200 MHz, CHLOROFORM-d) δ ppm 3.70 (d, J=4.8 Hz, 2 H) 3.86-4.02 (m, 2 H) 4.09-4.22 (m, 2 H) 4.40-4.68 (m, 7 H) 4.83 (d, J=11.4 Hz, 1 H) 7.12-7.41 (m, 20 H).

### Reference Example 2

Preparation of 2-[4-(benzyloxy)-5-bromo-2-methylphenyl]-1,3-dioxolane (compound (6A))

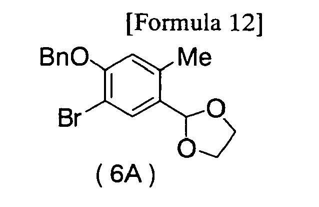

### (1) Preparation of 1-[4-(benzyloxy)-2-methylphenyl]ethanone

To a solution of 4'-hydroxy-2'-methylacetophenone (3.06 g, 20 mmol) in N,N-dimethylformamide (20 mL), potassium carbonate (3.66 g, 26.4 mmol), benzyl bromide (2.7 mL, 22.4 mmol) and n-Bu₄NI (0.75 g, 2.03 mmol) were added and stirred at room temperature for 14 hours. The reaction mixture was diluted with saturated aqueous ammonium chloride under ice cooling, followed by addition of water and ethyl acetate to separate the organic layer. The organic layer was washed with 20 wt.% aqueous sodium thiosulfate and brine, and then dried over anhydrous magnesium sulfate. After filtering off the desiccant, the solvent was distilled off under reduced pressure and the resulting residue was purified by silica gel column chromatography (hexane:ethyl acetate = 8:1 → 6:1) to give the titled compound (5.05 g, quant.) as a colorless powder.
¹H NMR (300 MHz, CHLOROFORM-d) δ ppm 2.55 (s, 3 H) 2.57 (s, 3 H) 5.11 (s, 2 H) 6.78-6.86 (m, 2 H) 7.30-7.47 (m, 5 H) 7.75 (dd, J=7.93, 1.09 Hz, 1 H).

### (2) Preparation of 4-(benzyloxy)-5-bromo-2-methylbenzoic acid

To a solution of 1-[4-(benzyloxy)-2-methylphenyl]ethanone (20.9 g, 87.1 mmol) in acetone (300 mL), a solution of NaBr (9.86 g, 95.9 mmol) in water (100 mL), water (200 mL) and Oxone® (oxone monopersulfate compound, Aldrich) (59.0 g, 95.9 mmol) were added and stirred at room temperature for 2.5 hours. The reaction mixture was mixed with a solution of sodium sulfite (20 g) in water (50 mL) under ice cooling, followed by addition of water and ethyl acetate to separate the organic layer. The organic layer was washed with 20 wt.% aqueous sodium sulfite and brine, and then dried over anhydrous magnesium sulfate. After filtering off the desiccant, the solvent was distilled off under reduced pressure to give a mixture (27.2 g) of 1-[4-(benzyloxy)-5-bromo-2-methylphenyl]ethanone and 1-[4-(benzyloxy)-3-bromo-2-methylphenyl]ethanone. To this mixture, a 5% solution of sodium hypochlorite (300 mL, 255 mmol) and a solution of potassium hydroxide (4.80 g, 85.3 mmol) in water (10 mL) were added and stirred at 120°C for 1 hour. The reaction mixture was cooled to room temperature and filtered to collect the precipitated insoluble matter, to which 2N hydrochloric acid was then added. After extraction with ethyl acetate, the organic layer was washed with 2N hydrochloric acid and brine, and then dried over anhydrous magnesium sulfate. After filtering off the desiccant, the solvent was distilled off under reduced pressure and the resulting residue was washed with methanol to give the titled compound (16.6 g, 59%, 2 steps) as a colorless powder.
¹H NMR (300 MHz, DMSO-d₆) δ ppm 2.45-2.57 (m, 3 H) 5.28 (s, 2 H) 7.18 (s, 1 H) 7.31-7.54 (m, 5 H) 8.03 (s, 1 H) 12.83 (brs, 1 H).
ESI m/z = 319 (M-H), 321 (M+2-H).

### (3) Preparation of 2-[4-(benzyloxy)-5-bromo-2-methylphenyl]-1,3-dioxolane

To a suspension of 4-(benzyloxy)-5-bromo-2-methylbenzoic acid (16.6 g, 51.7 mmol) in chloroform (80 mL), oxalyl chloride (5 mL, 56.9 mmol)and N,N-dimethylformamide (6 drops) were added and stirred at room temperature for 1 hour. The reaction mixture was concentrated to give 4-(benzyloxy)-5-bromo-2-methylbenzoyl chloride. Next, to a suspension of N,O-dimethylhydroxylamine hydrochloride (5.55 g, 56.9 mmol) and triethylamine (15 mL, 103 mmol) in chloroform (60 mL), a solution of 4-(benzyloxy)-5-bromo-2-methylbenzoyl chloride in chloroform (60 mL) was added dropwise under ice cooling and stirred at room temperature for 1 hour, followed by addition of water and chloroform under ice cooling to separate the organic layer. The organic layer was washed with saturated aqueous sodium bicarbonate and brine, and then dried over anhydrous magnesium sulfate. After filtering off the desiccant, the solvent was distilled off under reduced pressure to give 4-(benzyloxy)-5-bromo-N-methoxy-N-methylbenzamide. To a solution of this product in tetrahydrofuran (150 mL), lithium aluminum hydride (1.96 g, 51.7 mmol) was added at -10°C and stirred at the same temperature for 1 hour. The reaction mixture was diluted with 1N hydrochloric acid, followed by addition of ethyl acetate to separate the organic layer. The organic layer was washed with 1N hydrochloric acid, saturated aqueous sodium bicarbonate and brine, and then dried over anhydrous magnesium sulfate. After filtering off the desiccant, the solvent was distilled off under reduced pressure to give 4-(benzyloxy)-5-bromo-2-methylbenzaldehyde. To a solution of this product in toluene (120 mL), ethylene glycol (30 mL, 517 mmol) and p-toluenesulfonic acid monohydrate (0.50 g, 2.58 mmol) were added and heated under reflux for 1.5 hours using a Dean-Stark apparatus. Ethyl acetate was added to the reaction mixture to separate the organic layer. The organic layer was washed with water, saturated aqueous sodium bicarbonate and brine, and then dried over anhydrous magnesium sulfate. After filtering off the desiccant, the solvent was distilled off under reduced pressure and the resulting residue was purified by silica gel column chromatography (hexane:ethyl acetate = 5:1)) and further purified by NH-type silica gel column chromatography (chloroform) to give the titled compound (12.8 g, 71%, 3 steps) as a colorless powder.
¹H NMR (300 MHz, CHLOROFORM-d) δ ppm 2.34 (s, 3 H) 3.92-4.19 (m, 4 H) 5.15 (s, 2 H) 5.87 (s, 1 H) 6.74 (s, 1 H) 7.27-7.51 (m, 5 H) 7.72 (s, 1 H).
ESI m/z = 348.

### Reference Example 3

Preparation of 2,3,4,6-tetra-O-benzyl-1-C-[2-(benzyloxy)-5-(1,3-dioxolan-2-yl)-4-methylphenyl]-5-thio-D-glucopyranose (compound 7A))

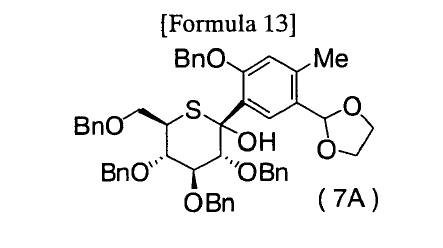

To a solution of 2-[4-(benzyloxy)-5-bromo-2-methylphenyl]-1,3-dioxolane (12.9 g, 36.9 mmol) in tetrahydrofuran (100 mL), 2.67 M n-butyllithium in hexane (14.5 mL, 36.9 mmol) was added dropwise at -78°C under a nitrogen atmosphere and stirred at the same temperature for 30 minutes. Then, a solution of 2,3,4,6-tetra-O-benzyl-5-thio-D-glucono-1,5-lactone (9.77 g, 17.6 mmol) in tetrahydrofuran (40 mL) was added dropwise and stirred at the same temperature for 15 minutes. After addition of saturated aqueous ammonium chloride, the reaction mixture was extracted with ethyl acetate. The organic layer was washed with saturated aqueous ammonium chloride and brine, and then dried over anhydrous magnesium sulfate. After filtering off the desiccant, the solvent was distilled off under reduced pressure and the resulting residue was purified by silica gel column chromatography (hexane:ethyl acetate = 3:1 → 2:1) to give the titled compound (10.6 g, 73%) as a colorless and transparent amorphous substance.
¹H NMR (300 MHz, CHLOROFORM-d) δ ppm 2.39 (s, 3 H) 3.46-3.72 (m, 2 H) 3.86-4.22 (m, 8 H) 4.43-5.00 (m, 8 H) 5.10 (s, 2 H) 5.92 (s, 1 H) 6.66-6.90 (m, 3 H) 7.00-7.38 (m, 23 H) 7.57 (brs, 1 H).
ESI m/z = 847 (M+Na⁺).

### Reference Example 4

Preparation of 2,3,4,6-tetra-O-benzyl-1-C-[2-(benzyloxy)-5-formyl-4-methylphenyl]-5-thio-D-glucopyranose (compound (8A))

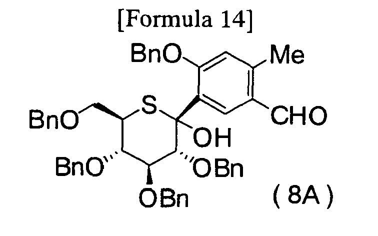

To a solution of 2,3,4,6-tetra-O-benzyl-1-C-[2-(benzyloxy)-5-(1,3-dioxolan-2-yl)-4-methylphenyl]-5-thio-D-glucopyranose (11.1 g, 13.5 mmol) in tetrahydrofuran (100 mL), 6N hydrochloric acid (100 mL) was added under ice cooling and stirred at room temperature for 12 hours. After addition of water under ice cooling, the reaction mixture was extracted with ethyl acetate. The organic layer was washed with saturated aqueous sodium bicarbonate and brine, and then dried over anhydrous magnesium sulfate. After filtering off the desiccant, the solvent was distilled off under reduced pressure and the resulting residue was purified by silica gel column chromatography (hexane:ethyl acetate = 2:1) to give the titled compound (10.1 g, quant.) as a light-yellow oily compound.
¹H NMR (300 MHz, CHLOROFORM-d) δ ppm 2.64 (s, 3 H) 3.51-3.70 (m, 2 H) 3.84-4.29 (m, 4 H) 4.46-4.97 (m, 8 H) 5.04-5.24 (m, 2 H) 6.62-6.82 (m, 3 H) 6.99-7.38 (m, 23 H) 7.60 (brs, 1 H) 10.05 (s, 1 H).
ESI m/z = 803 (M+Na⁺).

### Reference Example 5

Preparation of 1-(benzyloxy)-2-bromo-5-methyl-4-(4-methoxybenzyl)benzene

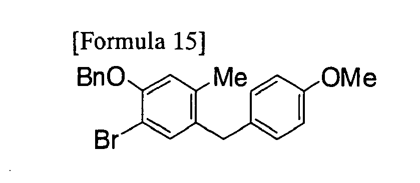

(1) To a solution of 4-(benzyloxy)-5-bromo-2-methylbenzaldehyde (3.0 g, 9.83 mmol) in tetrahydrofuran (20 mL), 0.5 M 4-methoxyphenylmagnesium bromide in tetrahydrofuran (29.5 mL, 14.7 mmol) was added dropwise at -18°C and stirred at -15°C for 15 minutes. After addition of saturated aqueous ammonium chloride, the reaction mixture was extracted with ethyl acetate. The organic layer was washed with saturated aqueous ammonium chloride and brine, and then dried over anhydrous magnesium sulfate. After filtering off the desiccant, the solvent was distilled off under reduced pressure and the resulting residue was purified by silica gel column chromatography (hexane:ethyl acetate = 4:1) to give [1-(benzyloxy)-2-bromo-5-methylphenyl](4-methoxyphenyl)methanol (4.48 g) as a colorless oily compound.

(2) Next, to a solution of [1-(benzyloxy)-2-bromo-5-methylphenyl](4-methoxyphenyl)methanol (4.40 g) in a mixed solvent of acetonitrile (20 mL) and chloroform (20 mL), Et₃SiH (3.1 mL, 19.7 mmol) and BF_{3·}Et₂O (1.2 mL, 9.83 mmol) were added sequentially at 4°C. After stirring at the same temperature for 30 minutes, 2M aqueous potassium hydroxide (20 mL) was added. The resulting mixture was extracted with chloroform, and the organic layer was washed with 1M hydrochloric acid and brine, and then dried over anhydrous magnesium sulfate. After filtering off the desiccant, the solvent was distilled off under reduced pressure and the resulting residue was purified by silica gel column chromatography (hexane:ethyl acetate = 10:1) to give the titled compound (3.46 g, 89%) as a colorless oil.
¹H NMR (300 MHz, CHLOROFORM-d) δ ppm 2.17 (s, 3 H) 3.79 (s, 3 H) 3.82 (s, 2 H) 5.12 (s, 2 H) 6.77 (s, 1 H) 6.82 (d, J=8.4 Hz, 2 H) 7.02 (d, J=8.4 Hz, 2 H) 7.19-7.45 (m, 4 H) 7.44-7.58 (m, 2 H).
EI m/z = 396, 398.

### Reference Example 6

Preparation of 1-(benzyloxy)-2-bromo-5-methyl-4-(4-methylbenzyl)benzene

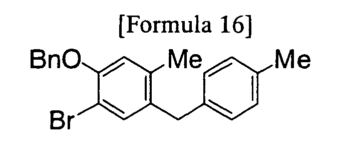

The same procedure as shown in Reference Example 5 was repeated to synthesize the titled compound, except that 4-methoxyphenylmagnesium bromide was replaced with 4-methylphenylmagnesium bromide.
¹H NMR (300 MHz, CHLOROFORM-d) δ ppm 2.17 (s, 3 H) 2.31 (s, 3 H) 3.84 (s, 2 H) 5.12 (s, 2 H) 6.76 (s, 1 H) 6.95-7.03 (m, 2 H) 7.04-7.14 (m, 2 H) 7.22-7.58 (m, 6H).
EI m/z = 380, 382.

### Reference Example 7

Preparation of 1-(benzyloxy)-2-bromo-4-(4-ethylbenzyl)-5-methylbenzene

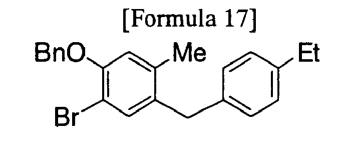

### (1) Preparation of [4-(benzyloxy)-5-bromo-2-methylphenyl](4-ethylphenyl)methanol

To a solution of 1-bromo-4-ethylbenzene (5.00 g, 16.4 mmol) in tetrahydrofuran (30 mL), 2.66 M n-BuLi in hexane (6.47 mL, 17.2 mmol) was added dropwise at -60°C over 5 minutes and stirred at the same temperature for 15 minutes. To this mixture, a solution of 4-(benzyloxy)-5-bromo-2-methylbenzaldehyde (3.03 g, 16.4 mmol) in tetrahydrofuran (15 mL) was added dropwise and stirred at the same temperature for 15 minutes. The reaction mixture was diluted with saturated aqueous ammonium chloride and warmed to room temperature, followed by extraction with ethyl acetate. The organic phase was washed with brine and then dried over anhydrous magnesium sulfate. After filtering off the desiccant, the solvent was distilled off under reduced pressure to give the titled compound.

### (2) Preparation of 1-(benzyloxy)-2-bromo-4-(4-ethylbenzyl)-5-methylbenzene

Next, to a solution of [4-(benzyloxy)-5-bromo-2-methylphenyl](4-ethylphenyl)methanol in chloroform (80 mL), Et₃SiH (3.93 mL, 24.6 mmol) and BF₃·Et₂O (2.49 mL, 19.7 mmol) were added sequentially at 0°C and stirred at the same temperature for 30 minutes. After addition of saturated aqueous sodium bicarbonate, the reaction mixture was extracted with ethyl acetate. The organic phase was washed with brine and then dried over anhydrous magnesium sulfate. After filtering off the desiccant, the solvent was distilled off under reduced pressure and the resulting residue was purified by silica gel column chromatography (hexane:ethyl acetate = 98:2) to give the titled compound (5.31 g, 82%, 2 steps) as a colorless oil.
¹H NMR (300 MHz, CHLOROFORM-d) δ ppm 1.22 (t, J=7.62 Hz, 3 H) 2.17 (s, 3 H) 2.61 (q, J=7.62 Hz, 2 H) 3.85 (s, 2 H) 5.12 (s, 2 H) 6.76 (s, 1 H) 7.01 (d, 2 H) 7.10 (d, 2 H) 7.27-7.43 (m, 4 H) 7.48 (d, 2 H).
ESI m/z = 412, 414 (M+NH₄⁺).

### Reference Example 8

Preparation of 1-(benzyloxy)-2-bromo-5-methyl-4-[4-(methylsulfanyl)benzyl]benzene 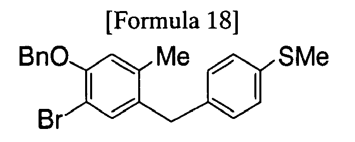 The same procedure as shown in Reference Example 7 was repeated to give [4-(benzyloxy)-5-bromo-2-methylphenyl][4-(methylsulfanyl)phenyl]methanol (6.93 g, 66%), except that 4-bromotoluene was replaced with 4-bromothioanisole.
¹H NMR (300 MHz, CHLOROFORM-d) δ ppm 2.05 (d, J=3.6 Hz, 1 H) 2.16 (s, 3 H) 2.47 (s, 3 H) 5.13 (s, 2 H) 5.86 (d, J=3.6 Hz, 1 H) 6.73 (s, 1 H) 7.22 (s, 4 H) 7.28-7.51 (m, 5 H) 7.70 (s, 1 H).
ESI m/z = 463 (M-Cl⁻).
Subsequently, [4-(benzyloxy)-5-bromo-2-methylphenyl](4-ethylphenyl)methanol was replaced with [4-(benzyloxy)-5-bromo-2-methylphenyl][4-(methylsulfanyl)phenyl]methanol to give the titled compound (6.28 g, 94%) as a light-brown oil.
¹H NMR (300 MHz, CHLOROFORM-d) δ ppm 2.15 (s, 3 H) 2.46 (s, 3 H) 3.84 (s, 2 H) 5.12 (s, 2 H) 6.77 (s, 1 H) 6.98-7.06 (m, 2 H) 7.14-7.21 (m, 2 H) 7.23-7.51 (m, 6 H).
ESI m/z = 430 (M+NH₄⁺).

### Reference Example 9

Preparation of 5-bromo-2-chloro-4-methoxybenzaldehyde

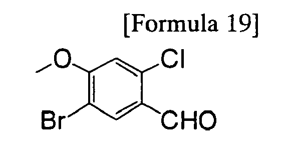

### (1) Preparation of 1-bromo-4-chloro-2-methoxy-5-methylbenzene

(i) To a solution of 4-chloro-2-methoxy-5-methylaniline (20.2 g, 117 mmol) in acetone (100 mL), 48% aqueous hydrogen bromide (50 mL, 2.93 moL) was added, and a solution of sodium nitrite (10.1 g, 146 mmol) in water (70 mL) was then added dropwise under ice cooling. The reaction mixture was warmed to room temperature and stirred for 1.5 hours.
(ii) To a solution of copper sulfate pentahydrate (87.9 g, 352 mmol) and sodium bromide (36.2 g, 352 mmol) in water (160 mL), a solution of sodium sulfite (22.4 g, 176 mmol) in water (70 mL) was added dropwise over 20 minutes. After stirring at room temperature for 30 minutes, the reaction mixtures was allowed to stand under ice cooling. The supernatant was removed by decantation and the resulting precipitate was further washed with water (1 L), followed by decantation of the supernatant (4 times) to give copper bromide as a colorless powder. To this powder, 48% aqueous hydrogen bromide (50 mL, 2.93 moL) was added and stirred, and the aqueous diazonium salt solution prepared in (i) was then added dropwise over 40 minutes under ice cooling. After stirring at room temperature for 15 hours, the reaction mixture was evaporated under reduced pressure to remove acetone and the resulting precipitate was extracted with ethyl acetate. The organic layer was washed with brine and then dried over anhydrous magnesium sulfate. After filtering off the desiccant, the solvent was distilled off under reduced pressure and the resulting residue was purified by silica gel column chromatography (hexane) to give the titled compound (23.5 g) as a colorless crystal.

### (2) Preparation of 1-bromo-(5-bromomethyl)-4-chloro-2-methoxybenzene

Next, to a solution of 1-bromo-4-chloro-2-methoxy-5-methylbenzene (23.2 g) in carbon tetrachloride (400 mL), N-bromosuccinimide (19.2 g) and 2,2'-azobis(2-methylpropionitrile) (1.61 g) were added and heated under reflux for 1 hour. After cooling, the reaction mixture was diluted with water and extracted with ethyl acetate. The organic layer was washed with brine and then dried over anhydrous magnesium sulfate. After filtering off the desiccant, the solvent was distilled off under reduced pressure to give the titled compound (33.5 g) as a colorless oil.

### (3) Preparation of (5-bromo-2-chloro-4-methoxyphenyl)methanol

To a solution of 1-bromo-(5-bromomethyl)-4-chloro-2-methoxybenzene (33.4 g) in 1,4-dioxane (350 mL), a solution of sodium carbonate (52.0 g) in water (350 mL) was added and heated under reflux for 1 hour. After cooling, the reaction mixture was diluted with water (350 mL) and extracted with ethyl acetate. The organic layer was washed with brine and then dried over anhydrous magnesium sulfate. After filtering off the desiccant, the solvent was distilled off under reduced pressure to give the titled compound (25.8 g) as a colorless powder.

### (4) Preparation of 5-bromo-2-chloro-4-methoxybenzaldehyde

To a solution of (5-bromo-2-chloro-4-methoxyphenyl)methanol (25.8 g) in chloroform (300 mL), manganese dioxide (129 g, 1.48 mol) was added and stirred at room temperature for 18 hours. After the insoluble matter was filtered off on celite, the solvent was distilled off under reduced pressure and the resulting residue was purified by silica gel column chromatography (hexane:ethyl acetate = 50:1 → 30:1) to give the titled compound (20.0 g, 82%, 4 steps) as a colorless powder.
¹H NMR (300 MHz, CHLOROFORM-d) δ ppm 3.99 (s, 3 H) 6.92 (s, 1 H) 8.12 (s, 1 H) 10.27 (s, 1 H).

### Reference Example 10

Preparation of 5-bromo-2-chloro-4-hydroxybenzaldehyde 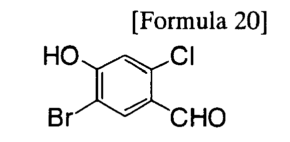 To a solution of 5-bromo-2-chloro-4-methoxybenzaldehyde (18.6 g, 74.4 mmol) in dimethyl sulfoxide (300 mL), pyridine hydrochloride (43.0 g, 372 mmol) was added and stirred at 145°C for 3 hours. The reaction mixture was cooled on ice, acidified with 10% aqueous hydrochloric acid and extracted with ethyl acetate. The organic layer was washed with brine and then dried over anhydrous magnesium sulfate. After filtering off the desiccant, the solvent was distilled off under reduced pressure to give a colorless crystal (20.1 g). This was recrystallized from chloroform to give the titled compound (11.7 g, 67%) as a colorless crystal.
¹H NMR (300 MHz, DMSO-d₆) δ ppm 7.07 (s, 1 H) 7.96 (s, 1 H) 10.08 (s, 1 H) 12.05 (brs, 1 H).

### Reference Example 11

Preparation of 5-bromo-2-chloro-4-(prop-2-en-1-yloxy)benzaldehyde 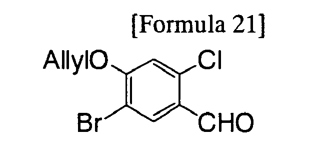 To a solution of 5-bromo-2-chloro-4-hydroxybenzaldehyde (12.0 g, 51.0 mmol), tetrabutylammonium iodide (941 mg, 2.55 mmol) and potassium carbonate (11.3 g, 81.5 mmol) in N,N-dimethylformamide (250 mL), allyl bromide (5.61 mL, 66.3 mmol) was added and stirred at room temperature for 16 hours. After cooling on ice, the reaction mixture was mixed with 10% aqueous hydrochloric acid (50 mL) and further with water (200 mL), followed by extraction with ethyl acetate. The organic layer was washed with brine and dried over anhydrous magnesium sulfate. After filtering off the desiccant, the solvent was distilled off under reduced pressure and the resulting residue was recrystallized from ethanol to give the titled compound (11.5 g, 82%) as a colorless powder.
¹H NMR (300 MHz, CHLOROFORM-d) δ ppm 4.70 (dt, J=5.0, 1.6 Hz, 2 H) 5.40 (dq, J=10.6, 1.4 Hz, 1 H) 5.52 (dd, J=17.3, 1.1 Hz, 1 H) 6.04 (dd, J=17.0, 10.3 Hz, 1 H) 6.90 (s, 1 H) 8.13 (s, 1 H) 10.27 (s, 1 H).

### Reference Example 12

Preparation of 1-bromo-4-chloro-5-(4-methoxybenzyl)-2-(prop-2-en-1-yloxy)benzene

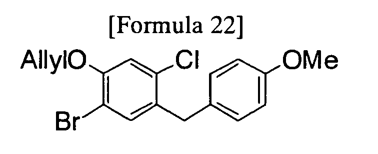

### (1) Preparation of [5-bromo-2-chloro-4-(prop-2-en-1-yloxy)phenyl](4-methoxyphenyl)methanol

To a solution of 1-bromo-4-methoxybenzene (1.43 mL, 11.43 mmol) in tetrahydrofuran (100 mL), 2.64 M n-BuLi in hexane (4.54 mL, 11.98 mmol) was added dropwise at -80°C over 5 minutes and stirred at the same temperature for 15 minutes. To this mixture, a solution of 5-bromo-2-chloro-4-(prop-2-en-1-yloxy)benzaldehyde (3.00 g, 10.89 mmol) in tetrahydrofuran (50 mL) was added dropwise. The resulting mixture was gradually warmed to room temperature and stirred at the same temperature for 30 minutes. The reaction mixture was diluted with saturated aqueous ammonium chloride and concentrated under reduced pressure to remove tetrahydrofuran. This was extracted with ethyl acetate, and the organic phase was washed with brine and then dried over anhydrous magnesium sulfate. After filtering off the desiccant, the solvent was distilled off under reduced pressure to give the titled compound (3.79 g) as a light-yellow oily compound.

### (2) Preparation of 1-bromo-4-chloro-5-(4-methoxybenzyl)-2-(prop-2-en-1-yloxy)benzene

Next, to a solution of [5-bromo-2-chloro-4-(prop-2-en-1-yloxy)phenyl](4-methoxyphenyl)methanol (3.77 g, 9.83 mmol) in chloroform (50 mL), Et₃SiH (2.35 mL, 14.74 mmol) and BF_{3·}Et₂O (1.49 mL, 11.79 mmol) were added sequentially at 0°C and stirred at the same temperature for 30 minutes. After addition of saturated aqueous sodium bicarbonate, the reaction mixture was extracted with ethyl acetate. The organic phase was washed with brine and then dried over anhydrous magnesium sulfate. After filtering off the desiccant, the solvent was distilled off under reduced pressure and the resulting residue was purified by silica gel column chromatography (chloroform:hexane = 1:4) to give the titled compound (1.56 g, 39%, 2 steps) as a colorless oily compound.
¹H NMR (300 MHz, CHLOROFORM-d) δ ppm 3.79 (s, 3 H) 3.93 (s, 2 H) 4.57 (dt, J=5.0, 1.6 Hz, 2 H) 5.32 (dd, J=10.6, 1.4 Hz, 1 H) 5.50 (d, J=1.6 Hz, 1 H) 5.91-6.15 (m, 1 H) 6.84 (d, J=8.7 Hz, 2 H) 6.90 (s, 1 H) 7.09 (d, J=8.7 Hz, 2 H) 7.30 (s, 1 H).
EI m/z = 366, 368.

### Reference Example 13

Preparation of 1-bromo-4-chloro-5-(4-methylbenzyl)-2-(prop-2-en-2-yloxy)benzene

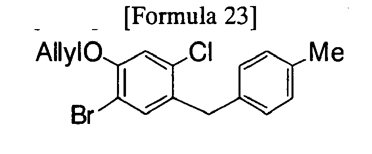

### (1) Preparation of [5-bromo-2-chloro-4-(prop-2-en-1-yloxy)phenyl](4-methylphenyl)methanol

The same procedure as shown in Reference Example 12(1) was repeated, except that 1-bromo-4-methoxybenzene was replaced with 4-bromotoluene. The resulting product was purified by silica gel column chromatography (hexane:ethyl acetate = 87:13) to give the titled compound (1.04 g, 38%) as a colorless solid.
¹H NMR (300 MHz, CHLOROFORM-d) δ ppm 2.21 (d, J=3.57 Hz, 1 H) 2.33 (s, 3 H) 4.55-4.61 (m, 2 H) 5.33 (dq, J=10.57, 1.45 Hz, 1 H) 5.47 (dq, J=17.31, 1.59 Hz, 1 H) 5.96-6.11 (m, 2 H) 6.84 (s, 1 H) 7.15 (d, 2 H) 7.25 (d, 2 H) 7.80 (s, 1 H).

### (2) Preparation of 1-bromo-4-chloro-5-(4-methylbenzyl)-2-(prop-2-en-2-yloxy)benzene

The same procedure as shown in Reference Example 12(2) was repeated, except that [5-bromo-2-chloro-4-(prop-2-en-1-yloxy)phenyl](4-methoxyphenyl)methanol was replaced with [5-bromo-2-chloro-4-(prop-2-en-1-yloxy)phenyl](4-methylphenyl)methanol. The resulting product was purified by silica gel column chromatography (hexane:ethyl acetate = 87:13) to give the titled compound (827 mg, 83%) as a colorless oil.
¹H NMR (300 MHz, CHLOROFORM-d) δ ppm 2.32 (s, 3 H) 3.96 (s, 2 H) 4.57 (dt, J=5.05, 1.59 Hz, 2 H) 5.32 (dq, J=10.57, 1.45 Hz, 1 H) 5.48 (dq, 1 H) 5.96-6.11 (m, J=17.23, 10.39, 5.15, 5.15 Hz, 1 H) 6.90 (s, 1 H) 7.05 (d, 2 H) 7.11 (d, 2 H) 7.31 (s, 1 H).
EI m/z = 350, 352.

### Reference Example 14

Preparation of 1-bromo-4-chloro-5-(4-ethylbenzyl)-2-(prop-2-en-1-yloxy)benzene

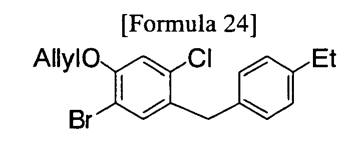

### (1) Preparation of [5-bromo-2-chloro-4-(prop-2-en-1-yloxy)phenyl](4-ethylphenyl)methanol

The same procedure as shown in Reference Example 12(1) was repeated, except that 1-bromo-4-methoxybenzene was replaced with 1-bromo-4-ethylbenzene. The resulting product was purified by silica gel column chromatography (hexane:ethyl acetate = 87:13) to give the titled compound (1.96 g, 35%) as a colorless solid.
¹H NMR (300 MHz, CHLOROFORM-d) δ ppm 1.22 (t, J=7.54 Hz, 3 H) 2.20 (d, J=3.57 Hz, 1 H) 2.63 (q, J=7.67 Hz, 2 H) 4.58 (dd, J=5.05, 0.70 Hz, 2 H) 5.33 (dq, J=10.59, 1.44 Hz, 1 H) 5.48 (dq, J=17.25, 1.71, 1.55 Hz, 1 H) 5.95-6.15 (m, 2 H) 6.85 (s, 1 H) 7.17 (d, 2 H) 7.28 (d, 2 H) 7.82 (s, 1 H).

### (2) Preparation of 1-bromo-4-chloro-5-(4-ethylbenzyl)-2-(prop-2-en-2-yloxy)benzene

The same procedure as shown in Reference Example 12(2) was repeated, except that [5-bromo-2-chloro-4-(prop-2-en-1-yloxy)phenyl](4-methoxyphenyl)methanol was replaced with [5-bromo-2-chloro-4-(prop-2-en-1-yloxy)phenyl](4-ethylphenyl)methanol. The resulting product was purified by silica gel column chromatography (hexane:ethyl acetate = 87:13) to give the titled compound (1.55 g, 83%) as a colorless oil.
¹H NMR (300 MHz, CHLOROFORM-d) δ ppm 1.22 (t, J=7.54 Hz, 3 H) 2.62 (q, J=7.77 Hz, 2 H) 3.97 (s, 2 H) 4.58 (dt, J=4.97, 1.55 Hz, 2 H) 5.32 (dq, J=10.57, 1.45 Hz, 1 H) 5.48 (dq, J=17.25, 1.66 Hz, 1 H) 6.04 (dddd, J=17.25, 10.41, 5.13, 4.97 Hz, 1 H) 6.90 (s, 1 H) 7.08 (d, 2 H) 7.13 (d, 2 H) 7.33 (s, 1 H).
EI m/z = 364, 366.

### Example 1

### (1-A)

Preparation of 2,3,4,6-tetra-O-benzyl-1-C-[2-(benzyloxy)-4-methyl-5-(4-methylbenzyl)phenyl]-5-thio-D-glucopyranose

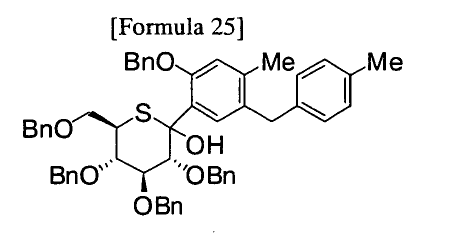

To a solution of 1-(benzyloxy)-2-bromo-5-methyl-4-(4-methylbenzyl)benzene (3.01 g, 7.89 mmol) in tetrahydrofuran (15 mL), 2.6 M n-BuLi in hexane (3.3 mL, 8.68 mmol) was added dropwise at -60°C over 4 minutes and stirred at the same temperature for 30 minutes. To this mixture, a solution of 2,3,4,6-tetra-O-benzyl-5-thio-D-glucono-1,5-lactone (2.91 g, 5.26 mmol) in tetrahydrofuran (10 mL) was added dropwise and stirred at the same temperature for 15 minutes. The reaction mixture was diluted with saturated aqueous ammonium chloride and warmed to room temperature, followed by extraction with ethyl acetate. The organic phase was washed with brine and then dried over anhydrous magnesium sulfate. After filtering off the desiccant, the solvent was distilled off under reduced pressure and the resulting residue was purified by silica gel column chromatography (hexane:ethyl acetate = 5:1) to give the titled compound (2.44 g, 54%) as a light-yellow gum.
¹H NMR (300 MHz, CHLOROFORM-d) δ ppm 2.20 (s, 3 H) 2.27 (s, 3 H) 3.46-3.59 (m, 1 H) 3.65 (dd, J=9.6, 2.4 Hz, 1 H) 3.78-4.16 (m, 5 H) 4.43-4.70 (m, 5 H) 4.75-4.97 (m, 4 H) 5.09 (s, 2 H) 6.70-7.42 (m, 31H).

### (1-B)

Preparation of (1S)-1,5-anhydro-2,3,4,6-tetra-O-benzyl-1-[2-(benzyloxy)-4-methyl-5-(4-methylbenzyl)phenyl]-1-thio-D-glucitol

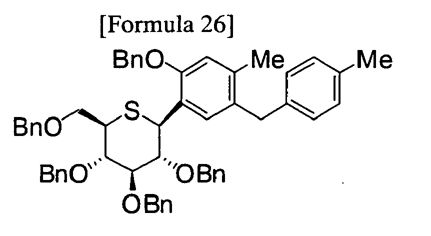

To a solution of 2,3,4,6-tetra-O-benzyl-1-C-[2-(benzyloxy)-4-methyl-5-(4-methylbenzyl)phenyl]-5-thio-D-glucopyranose (2.44 g, 2.85 mmol) in a mixed solvent of chloroform (5.0 mL) and acetonitrile (12.0 mL), Et₃SiH (0.91 mL, 5.69 mmol) and BF₃·Et₂O (0.43 mL, 3.42 mmol) were added sequentially at -15°C and stirred for 1 hour. After addition of saturated aqueous sodium bicarbonate, the reaction mixture was extracted with ethyl acetate. The organic phase was washed with brine and then dried over anhydrous magnesium sulfate. After filtering off the desiccant, the solvent was distilled off under reduced pressure and the resulting residue was purified by silica gel column chromatography (hexane:ethyl acetate = 8:1) to give the titled compound (2.1 g, 88%) as a colorless powder.

¹H NMR (300 MHz, CHLOROFORM-d) δ ppm 2.17 (s, 3 H) 2.25 (s, 3 H) 3.01-3.19 (m, 1 H) 3.49-3.60 (m, 1 H) 3.64-3.75 (m, 1 H) 3.77-3.97 (m, 5 H) 3.98-4.11 (m, 1 H) 4.48-4.66 (m, 5 H) 4.85 (s, 2 H) 4.90 (d, J=10.7 Hz, 1 H) 4.97-5.11 (m, 2 H) 6.66-7.52 (m, 31 H).
ESI m/z = 858 (M+NH₄⁺).

### Example 2

### (2-A)

Preparation of 2,3,4,6-tetra-O-benzyl-1-C-[2-(benzyloxy)-5-(4-methoxybenzyl)-4-methylphenyl]-5-thio-D-glucopyranose

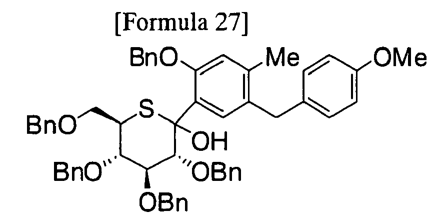

To a solution of 1-(benzyloxy)-2-bromo-4-(4-methoxybenzyl)-5-methylbenzene (3.46 g, 8.71 mmol) in tetrahydrofuran (17 mL), 2.6 M n-BuLi in hexane (3.7 mL, 9.58 mmol) was added dropwise at -50°C over 5 minutes and stirred at -60°C for 30 minutes. To this mixture, a solution of 2,3,4,6-tetra-O-benzyl-5-thio-D-glucono-1,5-lactone (3.22 g, 5.81 mmol) in tetrahydrofuran (10 mL) was added dropwise and stirred at the same temperature for 15 minutes. The reaction mixture was diluted with saturated aqueous ammonium chloride and warmed to room temperature, followed by extraction with ethyl acetate. The organic phase was washed with brine and then dried over anhydrous magnesium sulfate. After filtering off the desiccant, the solvent was distilled off under reduced pressure and the resulting residue was purified by silica gel column chromatography (hexane:ethyl acetate = 5:1) to give the titled compound (2.22 g, 44%) as a light-yellow gum.
¹H NMR (300 MHz, CHLOROFORM-d) δ ppm 2.20 (s, 3 H) 3.47-4.03 (m, 10 H) 4.02-4.21 (m, 2 H) 4.51 (s, 2 H) 4.63 (d, J=10.7 Hz, 1 H) 4.73-4.98 (m, 4 H) 5.10 (s, 2 H) 6.48-7.76 (m, 31 H).
ESI m/z = 895 (M+Na⁺).

### (2-B)

Preparation of (1S)-1,5-anhydro-2,3,4,6-tetra-O-benzyl-1-[2-(benzyloxy)-5-(4-methoxybenzyl)-4-methylphenyl]-1-thio-D-glucitol

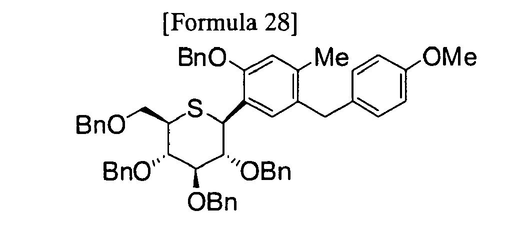

To a solution of 2,3,4,6-tetra-O-benzyl-1-C-[2-(benzyloxy)-5-(4-methoxybenzyl)-4-methylphenyl]-5-thio-D-glucopyranose (2.20 g, 2.52 mmol) in a mixed solvent of chloroform (6.0 mL) and acetonitrile (12.0 mL), Et₃SiH (0.80 mL, 5.04 mmol) and BF₃·Et₂O (0.38 mL, 3.02 mmol) were added sequentially at -15°C and stirred for 1 hour. After addition of saturated aqueous sodium bicarbonate, the reaction mixture was extracted with ethyl acetate. The organic phase was washed with brine and then dried over anhydrous magnesium sulfate. After filtering off the desiccant, the solvent was distilled off under reduced pressure and the resulting residue was purified by silica gel column chromatography (hexane:ethyl acetate = 5:1) to give the titled compound (2.15 g, 99%) as a colorless powder.

¹H NMR (300 MHz, CHLOROFORM-d) δ ppm 2.17 (s, 3 H) 3.00-3.23 (m, 1 H) 3.55 (t, J=10.8 Hz, 1 H) 3.66-4.16 (m, 10 H) 4.45-4.65 (m, 5 H) 4.85 (s, 2 H) 4.90 (d, J=10.7 Hz, 1 H) 4.96-5.13 (m, 2 H) 6.51-7.46 (m, 31 H).
ESI m/z = 879 (M+Na).

### Example 3

### (3-A)

Preparation of 2,3,4,6-tetra-O-benzyl-1-C-[2-(benzyloxy)-5-[(4-methoxyphenyl)(hydroxy)methyl]-4-methylphenyl]-5-thio-D-glucopyranose

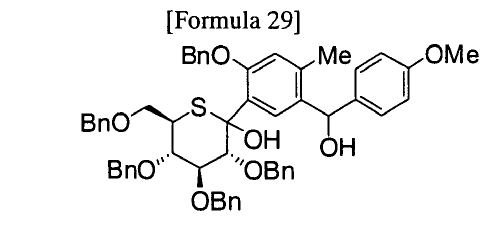

To a mixture of 1-bromo-4-methoxybenzene (520 mg, 2.78 mmol) and tetrahydrofuran (3 mL), 2.6 M n-BuLi in hexane (1.01 mL, 2.69 mmol) was added at -78°C. Immediately afterward, a solution of 2,3,4,6-tetra-O-benzyl-1-C-[2-(benzyloxy)-5-formyl-4-methylphenyl]-5-thio-D-glucopyranose (700 mg, 0.896 mmol) in tetrahydrofuran (3 mL) was added and further stirred for 30 minutes. The reaction mixture was warmed to room temperature, diluted with water and extracted with ethyl acetate. The organic phase was washed with brine and then dried over anhydrous magnesium sulfate. After filtering off the desiccant, the solvent was distilled off under reduced pressure and the resulting residue was purified by silica gel column chromatography (hexane:ethyl acetate = 2:1) to give the titled compound (570 mg, 72%) as a colorless amorphous substance.
¹H NMR (300 MHz, CHLOROFORM-d) δ ppm 2.03-2.24 (m, 3 H) 3.51-4.02 (m, 9 H) 4.49-5.16 (m, 10 H) 5.87 (brs, 1 H) 6.73-7.36 (m, 31 H).
ESI m/z = 911 (M+Na⁺), 887 (M-H).

### (3-B)

Preparation of (1S)-1,5-anhydro-2,3,4,6-tetra-O-benzyl-1-[2-(benzyloxy)-5-(4-methoxybenzyl)-4-methylphenyl]-1-thio-D-glucitol

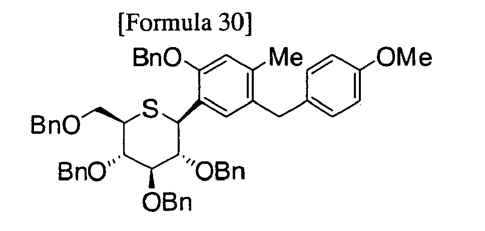

To a solution of 2,3,4,6-tetra-O-benzyl-1-C-[2-(benzyloxy)-5-[(4-methoxyphenyl)(hydroxy)methyl]-4-methylphenyl]-5-thio-D-glucopyranose (570 mg, 0.641 mmol) in acetonitrile (6.0 mL), Et₃SiH (0.308 mL, 1.93 mmol) and BF₃·Et₂O (0.090 mL, 1.41 mmol) were added sequentially at -10°C and stirred for 10 minutes. To the reaction mixture, chloroform (3.0 mL) was added and BF₃·Et₂O (0.090 mL, 1.41 mmol) was then added at 0°C. After stirring at 5°C for 30 minutes, the reaction mixture was diluted with saturated aqueous sodium bicarbonate and extracted with chloroform. The organic phase was washed with brine and then dried over anhydrous magnesium sulfate. After filtering off the desiccant, the solvent was distilled off under reduced pressure and the resulting residue was purified by silica gel column chromatography (hexane:ethyl acetate = 5:1) to give the titled compound (440 mg, 80%) as a colorless powder. The spectral data were identical with those obtained in Example 2.

### Example 4

Preparation of (1S)-1,5-anhydro-1-[4-methyl-5-(4-methylbenzyl)-2-hydroxyphenyl]-1-thio-D-glucitol (compound (I))

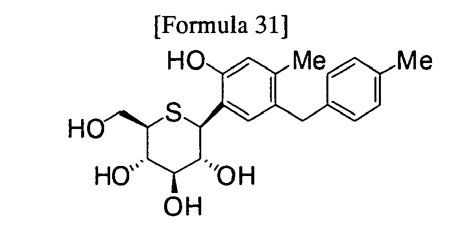

A mixture of (1S)-1,5-anhydro-2,3,4,6-tetra-O-benzyl-1-[2-(benzyloxy)-4-methyl-5-(4-methylbenzyl)phenyl]-1-thio-D-glucitol (2.1 g, 2.50 mmol) and 20% palladium hydroxide/activated carbon (2.06 g) in ethyl acetate (20 mL)-ethanol (20 mL) was stirred at room temperature for 50 hours under a hydrogen atmosphere. The reaction mixture was filtered through celite to remove the insoluble matter, and the filtrate was concentrated. The resulting residue was purified by silica gel column chromatography (chloroform:methanol = 10:1) to give the titled compound (340 mg, 35%) as a colorless powder.
¹H NMR (300 MHz, METHANOL-d₄) δ ppm 2.07 (s, 3 H) 2.27 (s, 3 H) 2.92-3.04 (m, 1 H) 3.58 (dd, J=10.3, 9.0 Hz, 1 H) 3.73 (dd, J=11.5, 6.6 Hz, 1 H) 3.78-3.88 (m, 3 H) 3.94 (dd, J=11.5, 3.8 Hz, 1 H) 4.29 (d, J=10.6 Hz, 1 H) 6.60 (s, 1 H) 6.94-6.98 (m, 2 H) 7.01-7.04 (m, 2 H) 7.05 (s, 1 H).
ESI m/z = 408 M+NH₄⁺), 389 (M-H).
Elementary analysis: Calcd for C₂₁H₂₆O₅S·H₂O: C, 61.72; H, 6.92. Found: C, 61.85; H, 6.78.

### Example 5

Preparation of (1S)-1,5-anhydro-1-[5-(4-methoxybenzyl)-4-methyl-2-hydroxyphenyl]-1-thio-D-glucitol (compound (II))

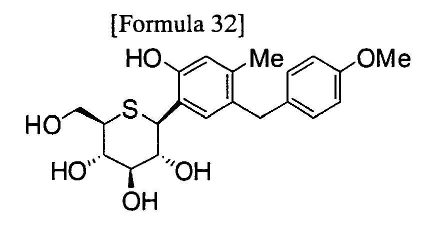

A mixture of (1S)-1,5-anhydro-2,3,4,6-tetra-O-benzyl-1-[2-(benzyloxy)-5-(4-methoxybenzyl)-4-methylphenyl]-1-thio-D-glucitol (2.11 g, 2.46 mmol) and 20% palladium hydroxide/activated carbon (2.1 g) in ethyl acetate (20 mL)-ethanol (20 mL) was stirred at room temperature for 24 hours under a hydrogen atmosphere. The reaction mixture was filtered through celite to remove the insoluble matter, and the filtrate was concentrated. The resulting residue was purified by silica gel column chromatography (chloroform:methanol = 10:1) to give the titled compound (690 mg, 69%) as a colorless powder.
¹H NMR (300 MHz, METHANOL-d₄) δ ppm 2.08 (s, 3 H) 2.91-3.06 (m, 1 H) 3.26 (t, 1 H) 3.59 (dd, J=10.3, 8.9 Hz, 1 H) 3.68-3.78 (m, 1 H) 3.74 (s, 3 H) 3.81 (s, 2 H) 3.82-3.88 (m, 1 H) 3.94 (dd, J=11.3, 3.7 Hz, 1 H) 4.29 (d, J=10.6 Hz, 1 H) 6.60 (s, 1 H) 6.69-6.82 (m, 2 H) 6.96-7.03 (m, 2 H) 7.04 (s, 1 H).
ESI m/z = 429 (M+Na⁺)
Elementary analysis: Calcd for C₂₁H₂₆O₆S·H₂O: C, 59.27; H, 6.65. Found: C, 59.32; H, 6.40.

### Example 6

Preparation of (1S)-1,5-anhydro-2,3,4,6-tetra-O-benzyl-1-[2-(benzyloxy)-5-(4-ethylbenzyl)-4-methylphenyl]-1-thio-D-glucitol

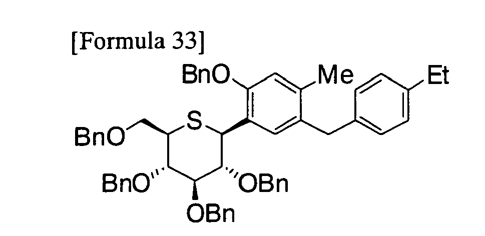

To a solution of 1-(benzyloxy)-2-bromo-4-(4-ethylbenzyl)-5-methylbenzene (5.31 g, 13.4 mmol) in tetrahydrofuran (50 mL), 2.66 M n-BuLi in hexane (5.05 mL, 13.4 mmol) was added dropwise at -60°C over 5 minutes and stirred at the same temperature for 15 minutes. To this mixture, a solution of 2,3,4,6-tetra-O-benzyl-5-thio-D-glucono-1,5-lactone (6.76 g, 12.2 mmol) in tetrahydrofuran (25 mL) was added dropwise and stirred at the same temperature for 2 hours. The reaction mixture was diluted with saturated aqueous ammonium chloride and warmed to room temperature, followed by extraction with ethyl acetate. The organic phase was washed with brine and then dried over anhydrous magnesium sulfate. After filtering off the desiccant, the solvent was distilled off under reduced pressure to give 2,3,4,6-tetra-O-benzyl-1-C-[2-(benzyloxy)-5-(4-ethylbenzyl)-4-methylphenyl]-5-thio-D-glucopyranose. This was dissolved in a mixed solvent of chloroform (30 mL) and acetonitrile (30 mL), and then cooled to 0°C. Next, Et₃SiH (2.92 mL, 18.3 mmol) and BF₃·Et₂O (1.86 mL, 14.6 mmol) were added sequentially and stirred for 1 hour. After addition of saturated aqueous sodium bicarbonate, the reaction mixture was extracted with ethyl acetate. The organic phase was washed with brine and then dried over anhydrous magnesium sulfate. After filtering off the desiccant, the solvent was distilled off under reduced pressure and the resulting residue was purified by NH-type silica gel column chromatography (hexane:ethyl acetate = 4:1) and silica gel column chromatography (hexane:ethyl acetate = 87:13) to give the titled compound (3.41 g, 33%, 2 steps) as a light-yellow gum.
¹H NMR (300 MHz, CHLOROFORM-d) δ ppm 1.16 (t, J=8.16 Hz, 3 H) 2.18 (s, 3 H) 2.48-2.61 (m, 2 H) 3.06-3.17 (m, 1 H) 3.54 (t, J=8.70 Hz, 1 H) 3.70 (d, J=10.26 Hz, 1 H) 3.76-3.98 (m, 5 H) 4.05 (d, J=11.81 Hz, 1 H) 4.50-4.64 (m, 5 H) 4.82-4.93 (m, 3 H) 5.00-5.10 (m, 2 H) 6.73 (s, 1 H) 6.78 (d, J=6.06 Hz, 2 H) 6.94 (br. s., 3 H) 7.09-7.19 (m, 5 H) 7.22-7.34 (m, 18 H) 7.35-7.44 (m, 2 H).
ESI m/z = 872 (M+NH₄⁺), 889 (M+Cl⁻).

### Example 7

Preparation of (1S)-1,5-anhydro-1-[5-(4-ethylbenzyl)-2-hydroxy-4-methylphenyl]-1-thio-D-glucitol (compound (III)) 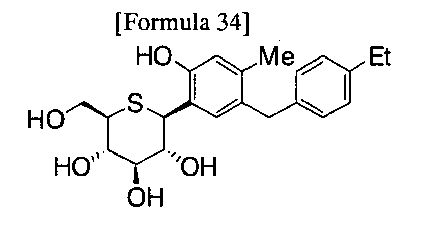 The same procedure as shown in Example 4 was repeated, except that (1S)-1,5-anhydro-2,3,4,6-tetra-O-benzyl-1-[2-(benzyloxy)-4-methyl-5-(4-methylbenzyl)phenyl]-1-thio-D-glucitol was replaced with (1S)-1,5-anhydro-2,3,4,6-tetra-O-benzyl-1-[2-(benzyloxy)-5-(4-ethylbenzyl)-4-methylphenyl]-1-thio-D-glucitol. The resulting product was purified by silica gel column chromatography, followed by recrystallization from ethanol to give the titled compound (392 mg, 24%) as a colorless powder.
¹H NMR (300 MHz, METHANOL-d4) δ ppm 1.19 (t, J=7.62 Hz, 3 H) 2.08 (s, 3 H) 2.58 (q, J=7.56 Hz, 2 H) 2.99 (ddd, J=10.22, 6.41, 3.73 Hz, 1 H) 3.25 (t, 1 H) 3.59 (dd, J=9.71, 8.47 Hz, 1 H) 3.74 (dd, J=11.50, 6.37 Hz, 1 H) 3.85 (t, J=9.64 Hz, 3 H) 3.95 (dd, J=11.42, 3.81 Hz, 1 H) 4.30 (d, J=10.41 Hz, 1 H) 6.61 (s, 1 H) 6.96-7.09 (m, 5 H).
ESI m/z = 422 (M+NH₄⁺), 403 (M-H), 439 (M+Cl⁻).

### Example 8

Preparation of (1S)-1,5-anhydro-2,3,4,6-tetra-O-benzyl-1-[2-(benzyloxy)-4-methyl-5-[4-(methylsulfanyl)benzyl]phenyl]-1-thio-D-glucitol

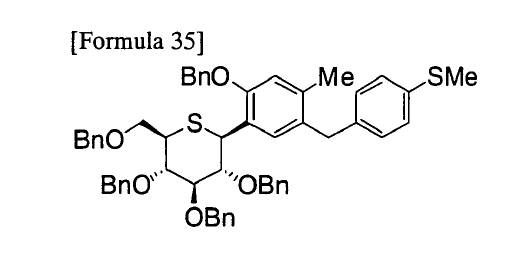

The same procedure as shown in Example 6 was repeated to give the titled compound (3.27 g, 27%, 2 steps) as a light-yellow gum, except that 1-(benzyloxy)-2-bromo-4-(4-ethylbenzyl)-5-methylbenzene was replaced with 1-(benzyloxy)-2-bromo-5-methyl-4-[4-(methylsulfanyl)benzyl]benzene.
¹H NMR (300 MHz, CHLOROFORM-d) δ ppm 2.16 (s, 3 H) 2.39 (s, 3 H) 3.07-3.16 (m, 1 H) 3.49-3.60 (m, 1 H) 3.63-3.75 (m, 1 H) 3.76-4.10 (m, 6 H) 4.42-4.66 (m, 5 H) 4.85 (s, 2 H) 4.90 (d, J=10.6 Hz, 1 H) 4.97-5.11 (m, 2 H) 6.69-7.48 (m, 31 H).

### Example 9

Preparation of (1S)-1,5-anhydro-1-[2-hydroxy-4-methyl-5-[4-(methylsulfanyl)benzyl]phenyl]-1-thio-D-glucitol (compound IV)) 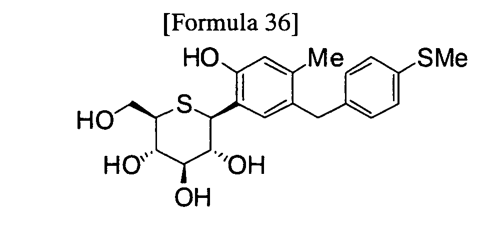 To a mixture of (1S)-1,5-anhydro-2,3,4,6-tetra-O-benzyl-1-[2-(benzyloxy)-4-methyl-5-[4-(methylsulfanyl)benzyl]phenyl]-1-thio-D-glucitol (1.09 g, 1.25 mmol), dimethyl sulfide (4.5 mL), m-cresol (1.2 mL), trifluoroacetic acid (7.5 mL) and 1,2-ethanedithiol (0.3 mL), trifluoromethanesulfonic acid (1.5 mL) was added dropwise at -20°C. After stirring at the same temperature for 40 minutes, the reaction mixture was poured into a mixture of saturated aqueous sodium carbonate and ice. After extraction with ethyl acetate, the organic phase was washed sequentially with saturated aqueous sodium carbonate and brine, and then dried over anhydrous magnesium sulfate. The solvent was distilled off under reduced pressure and the resulting residue was purified by silica gel column chromatography (chloroform:methanol = 50:1 → 10:1) and further recrystallized from ethanol to give the titled compound (166 mg, 31 %) as a colorless powder.
¹H NMR (300 MHz, METHANOL-d₄) δ ppm 2.08 (s, 3 H) 2.43 (s, 3 H) 2.99 (ddd, J=10.2, 6.4, 3.7 Hz, 1 H) 3.22-3.31 (m, 1 H) 3.59 (dd, J=10.3, 9.0 Hz, 1 H) 3.74 (dd, J=11.5, 6.4 Hz, 1 H) 3.80-3.88 (m, 3 H) 3.95 (dd, J=11.5, 3.7 Hz, 1 H) 4.30 (d, J=10.4 Hz, 1 H) 6.61 (s, 1 H) 6.99-7.08 (m, 3 H) 7.11-7.18 (m, 2 H).
ESI m/z = 440 (M+NH₄⁺), 445 (M+Na⁺), 421 (M-H), 457 (M+Cl⁻).

### Example 10

### (10-A)

Preparation of 1-C-[4-chloro-5-(4-methylbenzyl)-2-(prop-2-en-1-yloxy)phenyl]-2,3,4,6-tetra-O-prop-2-en-1-yl-5-thio-D-glucopyranose 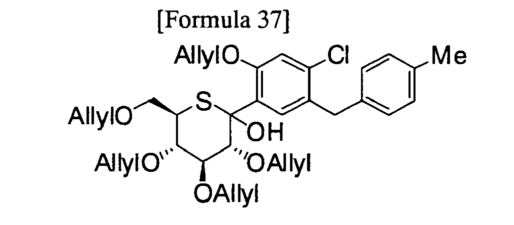 The same procedure as shown in Example 1(1-A) was repeated to give the titled compound (426 mg, 29%) as a colorless oil, except that 1-(benzyloxy)-2-bromo-5-methyl-4-(4-methylbenzyl)benzene was replaced with 1-bromo-4-chloro-5-(4-methylbenzyl)-2-(prop-2-en-2-yloxy)benzene, and 2,3,4,6-tetra-O-benzyl-5-thio-D-glucono-1,5-lactone was replaced with 2,3,4,6-tetra-O-prop-2-en-1-yl-5-thio-D-glucono-1,5-lactone.
¹H NMR (300 MHz, CHLOROFORM-d) δ ppm 2.29 (s, 3 H) 3.34-3.47 (m, 1 H) 3.47-4.31 (m, 14 H) 4.37 (dd, J=12.43, 5.75 Hz, 1 H) 4.60 (d, J=4.82 Hz, 2 H) 4.88 (s, 1 H) 4.94 (d, J=1.55 Hz, 1 H) 5.08-5.38 (m, 7 H) 5.42-5.59 (m, 2 H) 5.78-6.15 (m, 4 H) 6.93 (s, 1 H) 6.98-7.08 (m, 4 H) 7.24 (br. s., 1 H).
ESI m/z = 644 (M+NH₄⁺), 625 (M-H), 661 (M+Cl⁻).

### (10-B)

Preparation of (1S)-1,5-anhydro-1-[4-chloro-5-(4-methylbenzyl)-2-(prop-2-en-1-yloxy)phenyl]-2,3,4,6-tetra-O-prop-2-en-1-yl-1-thio-D-glucitol 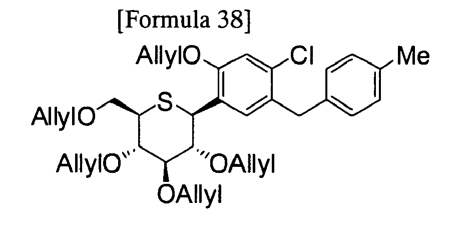 The same procedure as shown in Example 1(1-B) was repeated to give the titled compound (271 mg, 65%) as a colorless solid, except that 2,3,4,6-tetra-O-benzyl-1-C-[2-(benzyloxy)-4-methyl-5-(4-methylbenzyl)phenyl]-5-thio-D-glucopyranose was replaced with 1-C-[4-chloro-5-(4-methylbenzyl)-2-(prop-2-en-1-yloxy)phenyl]-2,3,4,6-tetra-O-prop-2-en-1-yl-5-thio-D-glucopyranose.
¹H NMR (300 MHz, CHLOROFORM-d) δ ppm 2.29 (s, 3 H) 2.94-3.04 (m, 1 H) 3.25 (t, J=9.09 Hz, 1 H) 3.38-4.20 (m, 11 H) 4.28 (d, J=5.60 Hz, 2 H) 4.36 (dd, J=12.05, 5.98 Hz, 2 H) 4.54 (dt, J=4.97, 1.63 Hz, 2 H) 4.81-4.92 (m, 2 H) 5.09-5.32 (m, 7 H) 5.33-5.51 (m, 2 H) 5.80-6.10 (m, 4 H) 6.86 (s, 1 H) 7.02 (td, 2 H) 7.03 (d, 2 H) 7.22 (s, 1 H).
ESI m/z = 628 (M+NH₄⁺), 633 (M+Na⁺), 645 (M+Cl⁻).

### Example 11

Preparation of (1S)-1,5-anhydro-1-[4-chloro-2-hydroxy-5-(4-methylbenzyl)phenyl]-1-thio-D-glucitol (compound (V)) 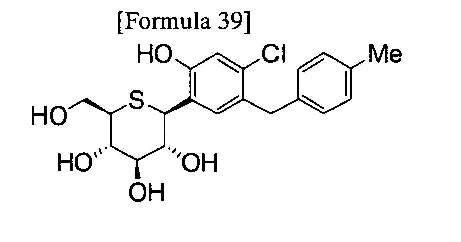 To a solution of (1S)-1,5-anhydro-1-[4-chloro-5-(4-methylbenzyl)-2-(prop-2-en-1-yloxy)phenyl]-2,3,4,6-tetra-O-prop-2-en-1-yl-1-thio-D-glucitol (242 mg, 0.396 mmol) in tetrahydrofuran (8 mL), N,N-dimethylbarbituric acid (618 mg, 3.96 mmol) and tetrakis(triphenylphosphine)palladium (91.5 mg, 0.0792 mmol) were added and stirred at 90°C for 3 hours. The reaction mixture was cooled to room temperature and water was added thereto, followed by extraction with ethyl acetate. The organic phase was washed with brine and then dried over anhydrous magnesium sulfate. After filtering off the desiccant, the solvent was distilled off under reduced pressure and the resulting residue was purified by silica gel column chromatography (chloroform:methanol = 9:1) and further recrystallized from hexane:ethyl acetate:ethanol to give the titled compound (64.2 mg, 39%) as a light-red powder.
¹H NMR (600 MHz, METHANOL-d₄) δ ppm 2.26 (s, 3 H) 2.95 (ddd, J=10.09, 6.42, 3.67 Hz, 1 H) 3.22 (t, J=8.71 Hz, 1 H) 3.53 (t, 1 H) 3.67-3.79 (m, 2 H) 3.84-3.96 (m, 3 H) 4.25 (d, J=10.55 Hz, 1 H) 6.80 (s, 1 H) 7.01 (d, 2 H) 7.03 (d, 2 H) 7.12 (s, 1 H).
ESI m/z = 428 (M+NH₄⁺), 433 (M+Na⁺), 409 (M-H), 445 (M+Cl⁻).

### Example 12

### (12-A)

Preparation of 1-C-[4-chloro-5-(4-ethylbenzyl)-2-(prop-2-en-1-yloxy)phenyl]-2,3,4,6-tetra-O-prop-2-en-1-yl-5-thio-D-glucopyranose 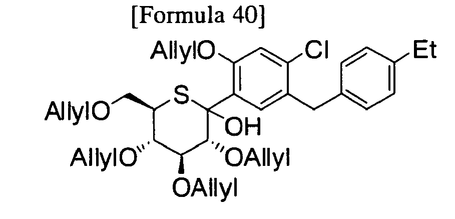 The same procedure as shown in Example 1(1-A) was repeated to give the titled compound (1.00 g, 39%) as a colorless oil, except that 1-(benzyloxy)-2-bromo-5-methyl-4-(4-methylbenzyl)benzene was replaced with 1-bromo-4-chloro-5-(4-ethylbenzyl)-2-(prop-2-en-2-yloxy)benzene, and 2,3,4,6-tetra-O-benzyl-5-thio-D-glucono-1,5-lactone was replaced with 2,3,4,6-tetra-O-prop-2-en-1-yl-5-thio-D-glucono-1,5-lactone.
¹H NMR (300 MHz, CHLOROFORM-d) δ ppm 1.20 (t, J=7.62 Hz, 3 H) 2.59 (q, J=7.51 Hz, 2 H) 3.37-3.46 (m, 1 H) 3.47-4.42 (m, 15 H) 4.60 (d, J=4.35 Hz, 2 H) 4.87 (s, 1 H) 4.92 (d, J=3.89 Hz, 1 H) 5.08-5.37 (m, 7 H) 5.41-5.58 (m, 2 H) 5.79-6.14 (m, 4 H) 6.93 (s, 1 H) 7.06 (d, 2 H) 7.07 (d, 2 H) 7.26 (br. s., 1 H).
ESI m/z = 639 (M-H), 675 (M+Cl⁻).

### (12-B)

Preparation of (1S)-1,5-anhydro-1-[4-chloro-5-(4-ethylbenzyl)-2-(prop-2-en-1-yloxy)phenyl]-2,3,4,6-tetra-O-prop-2-en-1-yl-1-thio-D-glucitol 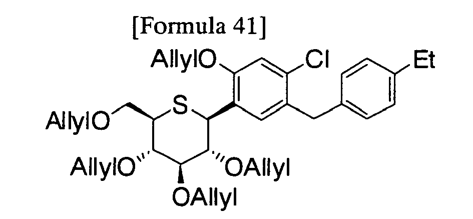 The same procedure as shown in Example 1(1-B) was repeated to give the titled compound (564 mg, 65%) as a colorless solid, except that 2,3,4,6-tetra-O-benzyl-1-C-[2-(benzyloxy)-4-methyl-5-(4-methylbenzyl)phenyl]-5-thio-D-glucopyranose was replaced with 1-C-[4-chloro-5-(4-ethylbenzyl)-2-(prop-2-en-1-yloxy)phenyl]-2,3,4,6-tetra-O-prop-2-en-1-yl-5-thio-D-glucopyranose.
¹H NMR (300 MHz, CHLOROFORM-d) δ ppm 1.20 (t, J=7.54 Hz, 3 H) 2.59 (q, J=7.82 Hz, 2 H) 2.95-3.04 (m, 1 H) 3.25 (t, J=8.55 Hz, 1 H) 3.36-3.94 (m, 7 H) 3.99 (d, J=5.60 Hz, 2 H) 4.03-4.19 (m, 2 H) 4.27 (d, J=5.75 Hz, 2 H) 4.36 (dd, J=12.43, 5.44 Hz, 2 H) 4.54 (dt, J=4.82, 1.63 Hz, 2 H) 4.85 (dd, J=15.85, 2.64 Hz, 2 H) 5.06-5.32 (m, 7 H) 5.32-5.48 (m, 2 H) 5.80-6.10 (m, 4 H) 6.86 (s, 1 H) 7.06 (d, J=3.26 Hz, 4 H) 7.22 (br. s., 1 H).
ESI m/z = 642 (M+NH₄⁺), 647 (M+Na⁺), 659 (M+Cl⁻).

### Example 13

Preparation of (1S)-1,5-anhydro-1-[4-chloro-2-hydroxy-5-(4-ethylbenzyl)phenyl]-1-thio-D-glucitol (compound (VI)) 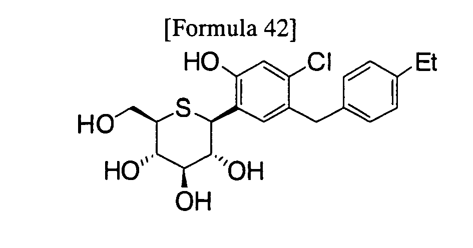 The same procedure as shown in Example 11 was repeated to give the titled compound (97.8 mg, 29%) as a light-red powder, except that (1S)-1,5-anhydro-1-[4-chloro-5-(4-methylbenzyl)-2-(prop-2-en-1-yloxy)phenyl]-2,3,4,6-tetra-O-prop-2-en-1-yl-1-thio-D-glucitol was replaced with (1S)-1,5-anhydro-1-[4-chloro-5-(4-ethylbenzyl)-2-(prop-2-en-1-yloxy)phenyl]-2,3,4,6-tetra-O-prop-2-en-1-yl-1-thio-D-glucitol.
¹H NMR (600 MHz, METHANOL-d₄) δ ppm 1.18 (t, J=7.57 Hz, 3 H) 2.57 (q, J=7.64 Hz, 2 H) 2.96 (ddd, J=10.20, 6.53, 3.44 Hz, 1 H) 3.22 (t, J=8.94 Hz, 1 H) 3.54 (dd, J=10.32, 8.94 Hz, 1 H) 3.71 (dd, J=11.69, 6.65 Hz, 1 H) 3.76 (dd, J=10.32, 8.94 Hz, 1 H) 3.86-3.96 (m, 3 H) 4.25 (d, J=10.55 Hz, 1 H) 6.80 (s, 1 H) 7.01-7.07 (m, 4 H) 7.14 (s, 1 H).
ESI m/z = 442 (M+NH₄⁺), 423 (M-H), 459 (M+Cl⁻).

### Example 14

### (14-A)

Preparation of 1-C-[4-chloro-5-(4-methoxybenzyl)-2-(prop-2-en-1-yloxy)phenyl]-2,3,4,6-tetra-O-prop-2-en-1-yl-5-thio-D-glucopyranose 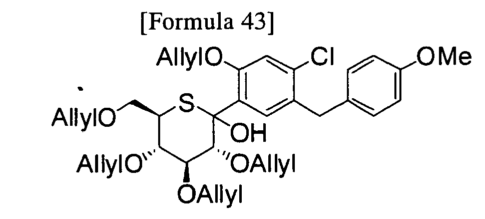 The same procedure as shown in Example 1(1-A) was repeated to give the titled compound (1.35 g, 54%) as a colorless oil, except that 1-(benzyloxy)-2-bromo-5-methyl-4-(4-methylbenzyl)benzene was replaced with 1-bromo-4-chloro-5-(4-methoxybenzyl)-2-(prop-2-en-1-yloxy)benzene, and 2,3,4,6-tetra-O-benzyl-5-thio-D-glucono-1,5-lactone was replaced with 2,3,4,6-tetra-O-prop-2-en-1-yl-5-thio-D-glucono-1,5-lactone.
¹H NMR (300 MHz, CHLOROFORM-d) δ ppm 3.35-3.47 (m, 1 H) 3.49-3.63 (m, 2 H) 3.69 (t, 2 H) 3.77 (s, 3 H) 3.80-4.42 (m, 10 H) 4.60 (d, J=5.0 Hz, 2 H) 4.82-4.99 (m, 2 H) 5.05-5.38 (m, 8 H) 5.41-5.60 (m, 2 H) 5.76-6.17 (m, 4 H) 6.73-6.84 (m, 2 H) 6.92 (s, 1 H) 6.99-7.11 (m, 2 H) 7.34 (brs., 1 H).
ESI m/z = 660 (M+NH₄⁺), 641 (M-H).

### (14-B)

Preparation of (1S)-1,5-anhydro-1-[4-chloro-5-(4-methoxybenzyl)-2-(prop-2-en-1-yloxy)phenyl]-2,3,4,6-tetra-O-prop-2-en-1-yl-1-thio-D-glucitol 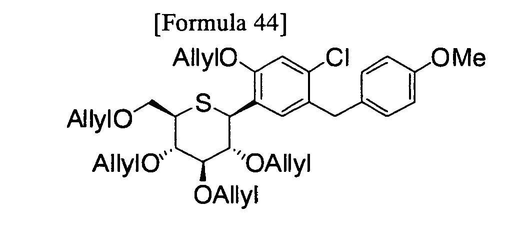 The same procedure as shown in Example 1(1-B) was repeated to give the titled compound (750 mg, 56%) as a colorless solid, except that 2,3,4,6-tetra-O-benzyl-1-C-[2-(benzyloxy)-4-methyl-5-(4-methylbenzyl)phenyl]-5-thio-D-glucopyranose was replaced with 1-C-[4-chloro-5-(4-methoxybenzyl)-2-(prop-2-en-1-yloxy)phenyl]-2,3,4,6-tetra-O-prop-2-en-1-yl-5-thio-D-glucopyranose.
¹H NMR (300 MHz, CHLOROFORM-d) δ ppm 2.88-3.08 (m, 1 H) 3.25 (t, J=9.0 Hz, 1 H) 3.35-4.66 (m, 20 H) 4.73-4.99 (m, 2 H) 5.06-5.33 (m, 7 H) 5.33-5.55 (m, 2 H) 5.72-6.18 (m, 4 H) 6.74-6.83 (m, 2 H) 6.86 (s, 1 H) 7.04 (d, J=8.7 Hz, 2 H) 7.21 (s, 1 H).
ESI m/z = 649 (M+Na⁺), 644 (M+NH₄⁺), 625 (M-H).

### Example 15

Preparation of (1S)-1,5-anhydro-1-[4-chloro-2-hydroxy-5-(4-methoxybenzyl)phenyl]-1-thio-D-glucitol (compound (VII)) 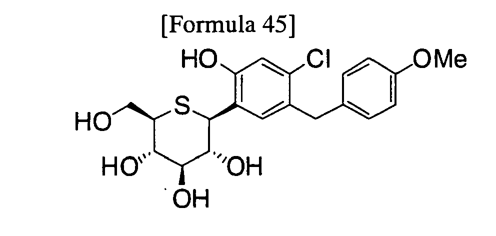 The same procedure as shown in Example 11 was repeated, except that (1S)-1,5-anhydro-1-[4-chloro-5-(4-methylbenzyl)-2-(prop-2-en-1-yloxy)phenyl]-2,3,4,6-tetra-O-prop-2-en-1-yl-1-thio-D-glucitol was replaced with (1S)-1,5-anhydro-1-[4-chloro-5-(4-methoxybenzyl)-2-(prop-2-en-1-yloxy)phenyl]-2,3,4,6-tetra-O-prop-2-en-1-yl-1-thio-D-glucitol. The resulting product was purified by silica gel column chromatography, followed by decoloring with activated carbon (powder) and recrystallization from ethyl acetate to give the titled compound (180 mg, 38%) as a colorless powder.
¹H NMR (300 MHz, METHANOL-d₄) δ ppm 2.91-3.05 (m, 1 H) 3.24 (t, J=8.9 Hz, 1 H) 3.29-3.35 (m, 2 H) 3.57 (t, J=9.6 Hz, 1 H) 3.66-3.87 (m, 4 H) 3.87-4.00 (m, 3 H) 4.27 (d, J=10.4 Hz, 1 H) 6.74-6.87 (m, 3 H) 7.01-7.11 (m, 2 H) 7.14 (s, 1 H).
ESI m/z = 449 (M+Na⁺), 444 (M+NH₄⁺), 425 (M-H).

Formulation Example

**[Table 1]**

| Formula for tablets containing 100 mg drug: | |
|---|---|
| Contents per tablet: | |
| Drug | 108.35 mg |
| Lactose monohydrate | 38.65 mg |
| Crystalline cellulose | 22.00 mg |
| Carboxymethylcellulose calcium | 20.00 mg |
| Hydroxypropylcellulose | 10.00 mg |
| Magnesium stearate | 1.00 mg |
| | 200.00 mg |

### Preparation procedure

A drug is mixed with lactose monohydrate, crystalline cellulose, carboxymethylcellulose calcium and hydroxypropylcellulose, followed by grinding in a mill. The ground mixture is mixed for 1 minute in a stirring granulator and then granulated with water for 4 to 8 minutes. The resulting granular product is dried at 70°C for 40 minutes. The dry granular powder is sieved through a 500 µm sieve. The sieved dry granular powder and magnesium stearate are mixed using a V-type blender at 30 rpm for 3 minutes. The granules for tabletting thus obtained are pressed and molded in a rotary tabletting machine to prepare tablets as indicated in Table 2.

**[Table 2]**

| |
|---|
| Tablet weight: 200 mg |
| Tablet size: 8 mm, round |

### Test Example 1

### (1) Cloning of human SGLT1 or human SGLT2 and its introduction into expression vector

A human SGLT1 sequence (NM_000343) was reverse-transcribed and amplified from human small intestine mRNA, and then introduced into pCMV-tag5A (Stratagene). Likewise, a human SGLT2 sequence (NM_003041) was prepared from human kidney mRNA in the same manner and introduced into pcDNA3.1+hygro (Invitrogen). The individual cloned sequences were confirmed to be identical with the sequences reported.

### (2) Creation of CHO-k1 cells stably expressing human SGLT1 or human SGLT2

The human SGLT1 and human SGLT2 expression vectors were each transfected into CHO-k1 cells using lipofectamine 2000 (Invitrogen). The cells were cultured in the presence of 500 µg/mL geneticin (SGLT1) or hygromycin B (SGLT2) to select resistant strains and specific activity of sugar uptake in the system shown below was used as an indicator to obtain SGLT-expressing cells.

### (3) Inhibition test for sodium-dependent sugar uptake in cells

The cells stably expressing human SGLT1 or human SGLT2 were used for an inhibition test of sodium-dependent sugar uptake activity.
The cells were incubated for 20 minutes in 1 mL pretreatment buffer (140 mM choline chloride, 2 mM KCI, 1 mM CaCl₂, 1 mM MgCl₂, 10 mM HEPES/5 mM Tris, pH 7.4). The pretreatment buffer was removed and replaced with 200 µL uptake buffer containing a test compound (methyl α-D-glucopyranoside (containing [¹⁴C]methyl α-D-glucopyranoside) at 0.1 mM for SGLT1 inhibition and 1 mM for SGLT2 inhibition, 140 mM NaCl, 2 mM KCI, 1 mM CaCl₂, 1 mM MgCl₂, 10 mM HEPES/5 mM Tris, pH 7.4). Uptake reaction was performed at 37°C for 30 minutes (SGLT1) or 1 hour (SGLT2). After the reaction, the cells were washed twice with 1 mL washing buffer (10 mM methyl α-D-glucopyranoside, 140 mM choline chloride, 2 mM KCI, 1 mM CaCl₂, 1 mM MgCl₂, 10 mM HEPES/5 mM Tris, pH 7.4), and then dissolved in a 0.2 M NaOH solution (400 µL). Aquazol 2 (Perkin Elmer) was added and mixed well with each sample, followed by measurement of radioactivity using a liquid scintillation counter (Beckman Coulter) to calculate the amount of sugar uptake. For the control group, uptake buffer containing no test compound was prepared. Moreover, another uptake buffer containing choline chloride instead of NaCl was also prepared for basal uptake.

Test compounds prepared at 6 appropriate concentrations were used to measure the amount of sugar uptake, and their concentrations required for 50% inhibition of the amount of sugar uptake were calculated as IC₅₀ values, assuming that the amount of sugar uptake in the control group was set to 100%.

### (4) Results

Table 3 shows SGLT inhibition activity measured for compounds (I) to (VII) and compound 98 (disclosed in Patent Document 9). Unexpectedly, compounds (I) to (VII), which were prepared by replacing the ethoxy group in compound 98 with a methyl group, an ethyl group, a methoxy group or a methylthio group, were found to show 3- to 6-fold stronger inhibitory activity against SGLT1 than compound 98, while retaining their inhibitory activity against SGLT2.

**[Table 3]**

| 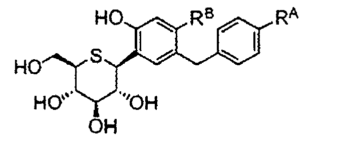 | | | IC₅₀ human SGLT1 (nM) | IC₅₀ human SGLT2 (nM) |
|---|---|---|---|---|
| Compound | R^{B} | R^{A} | | |
| I | Me | Me | 17 | 20 |
| II | Me | OMe | 32 | 20 |
| III | Me | Et | 32 | 12 |
| IV | Me | SMe | 27 | 10 |
| V | Cl | Me | 19 | 11 |
| VI | Cl | Et | 29 | 8 |
| VII | Cl | OMe | 33 | 13 |
| Compound 98 (disclosed in Document 9) | Me | OEt | 100 | 15 |

### (5) Test Example 2

### Confirmation test for hypoglycemic effect in db/db mice

The laboratory animals used were db/db mice (CLEA Japan, Inc., male, 7 weeks of age). A test substance was suspended in a 0.5% aqueous carboxymethylcellulose (CMC) solution and adjusted to a concentration of 1 mg/10 mL. On the day of the test, the db/db mice were divided into groups of 6 animals each, such that differences in the mean values and variance of their blood glucose levels were minimized, according to the procedures for blood collection and blood glucose measurement described later. The mice were measured for their body weight. Then, the test substance suspension was administered by oral gavage using a probe for oral administration at a volume of 10 mL/kg. The control group received a 0.5% aqueous CMC solution alone. Blood was collected at 8 points in total: before administration of the test substance (0 hour) and 0.5, 1, 2, 4, 6, 8 and 24 hours after oral administration. The test was performed under ad libitum feeding and drinking conditions.
Blood was collected from the orbital venous sinus of each animal under ether anesthesia using a heparin-coated blood collection tube and measured for its blood glucose level with a Glucose CII-Test Wako (Wako Pure Chemical Industries, Ltd., Japan). To determine the intensity (%) of hypoglycemic effect, casual blood glucose levels measured between 0 and 8 hours in each test group were analyzed by the trapezoidal method to calculate the area under the blood glucose-time curve (AUC). The results are expressed as a decrease in AUC relative to that of the control group.

### (6) Results

**[Table 4]**

| Compound | Intensity of hypoglycemic effect (%) |
|---|---|
| I | 48.5 |
| II | 49.7 |
| Compound 98 (disclosed in Document 9) | 35.0 |

### INDUSTRIAL APPLICABILITY

The present invention can be expected to provide a prophylactic or therapeutic agent for diabetes which comprises, as an active ingredient, a 1-phenyl 1-thio-D-glucitol compound inhibiting both SGLT1 (sodium-dependent glucose transporter 1) expressed in the small intestinal epithelium and SGLT2 (sodium-dependent glucose transporter 2) expressed in the kidney to achieve not only suppression of glucose absorption from the digestive tract but also excretion of urinary sugars.

## Claims

1. A 1-phenyl 1-thio-D-glucitol compound represented by formula (I): 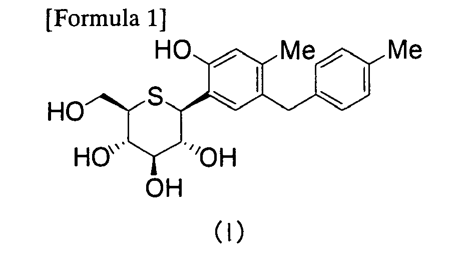 or a pharmaceutically acceptable salt thereof or a hydrate thereof.

2. A 1-phenyl 1-thio-D-glucitol compound represented by formula (II): 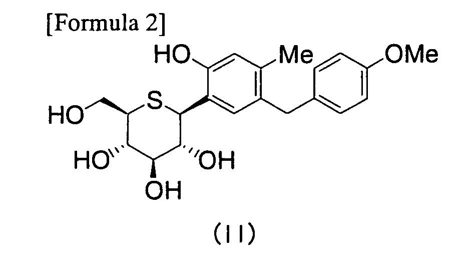 or a pharmaceutically acceptable salt thereof or a hydrate thereof.

3. A 1-phenyl 1-thio-D-glucitol compound represented by formula (III): 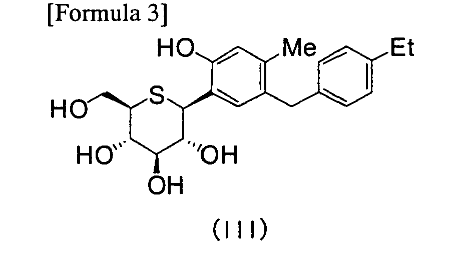 or a pharmaceutically acceptable salt thereof or a hydrate thereof.

4. A 1-phenyl 1-thio-D-glucitol compound represented by formula (IV): 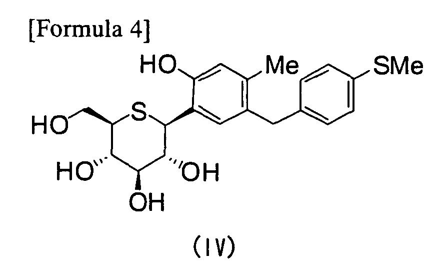 or a pharmaceutically acceptable salt thereof or a hydrate thereof.

5. A 1-phenyl 1-thio-D-glucitol compound represented by formula (V): 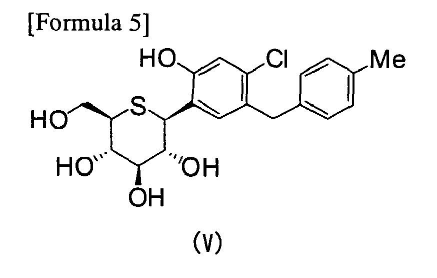 or a pharmaceutically acceptable salt thereof or a hydrate thereof.

6. A 1-phenyl 1-thio-D-glucitol compound represented by formula (VI): 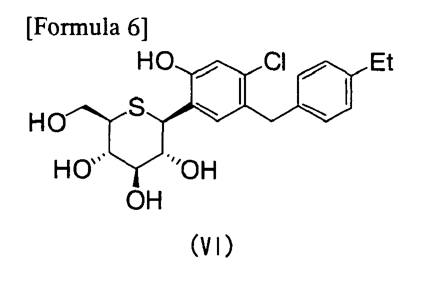 or a pharmaceutically acceptable salt thereof or a hydrate thereof.

7. A 1-phenyl 1-thio-D-glucitol compound represented by formula (VII): 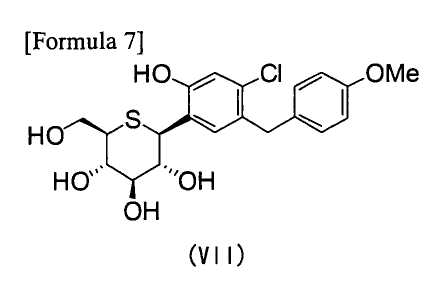 or a pharmaceutically acceptable salt thereof or a hydrate thereof.

8. A pharmaceutical preparation, which comprises the 1-phenyl 1-thio-D-glucitol compound according to any one of claims 1 to 7 or a pharmaceutically acceptable salt thereof or a hydrate thereof as an active ingredient.

9. The pharmaceutical preparation according to claim 8, which is an inhibitor of both sodium-dependent glucose transporter 1 activity and sodium-dependent glucose transporter 2 activity.

10. A prophylactic or therapeutic agent for diabetes, diabetes-related diseases or diabetic complications, which comprises the 1-phenyl 1-thio-D-glucitol compound according to any one of claims 1 to 7 or a pharmaceutically acceptable salt thereof or a hydrate thereof as an active ingredient.
